# EUROPÄISCHE PATENTANMELDUNG

(11) **EP 1 068 866 A2**
(43) Veröffentlichungstag der Anmeldung: **17.01.2001**
(21) Anmeldenummer: 00810580.1
(22) Anmeldetag: 04.07.2000
(51) Int. Cl.: A61K 7/48, A61K 7/135

(54) **Verwendung von Mischungen aus Mikropigmenten zur Bräunungsverhinderung und Aufhellung der Haut und Haare**

(30) Priorität: 12.07.1999 CH 128199
(71) Anmelder: Ciba Specialty Chemicals Holding Inc., 4057 Basel (CH)
(72) Erfinder: Fankhauser, Peter, 4107 Ettingen (CH); Luther, Helmut, 79639 Grenzach-Wyhlen (DE); Baschong, Werner, 4056 Basel (CH)

(57) **Zusammenfassung**

Beschrieben wird die Verwendung von mikronisierten organischen UV-Filtern zur Bräunungsverhinderung und Aufhellung der menschlichen Haut und Haare und ihre Verwendung in kosmetischen und in pharmazeutischen Formulierungen.

Die erfindungsgemäss verwendeten mikronisierten UV-Filter decken ein breites UV-Spektrum ab und besitzen daher ausgezeichnete Sonnenschutzeigenschaften.

## Beschreibung

Die vorliegende Erfindung betrifft die Verwendung von Mischungen aus mikronisierten organischen UV-Filtern zur Bräunungsverhinderung und Aufhellung der menschlichen Haut und Haare und ihre Verwendung in kosmetischen und in pharmazeutischen Formulierungen.

Es ist bekannt, dass bestimmte organische UV-Filter, wie z.B. schwerlösliche Benzotriazol- oder Triazinverbindungen ausgeprägte UV-Filtereigenschaften aufweisen, wenn sie in mikronisierter Form vorliegen.

Insbesondere in asiatischen Ländern besteht ein starkes Interesse an Lichtschutzfiltern oder Gemischen von Lichtschutzfiltern, die die Hautfarbe nach Sonneneinstrahlung erhalten und darüber hinaus in der Lage sind, der Haut ein helleres Aussehen zu verleihen.

Die Aufgabe der vorliegenden Erfindung besteht also darin, mikronisierte organische UV-Filter zu finden, die eine Bräunung der Haut verhindern und gleichzeitig die Haut aufhellen können.

Überraschenderweise wurde nun gefunden, dass mikronisierte organische UV-Filter oder Mischungen aus mindestens zwei mikronisierten UV-Filtern diese Aufgabe erfüllen können.

Gegenstand der vorliegenden Erfindung ist daher die Verwendung von Mischungen aus mikronisierten organischen UV-Filtern zur Bräunungsverhinderung und Aufhellung der menschlichen Haut.

Geeignete erfindungsgemäss verwendbare UV-Filter sind organische, z.T. schwerlösliche Verbindungen, wie z.B. Triazinderivate, insbesondere Hydroxyphenyltriazinverbindungen oder Benzotriazolderivate, eine Vinylgruppe enthaltende Amide, Zimtsäurederivate, sulfonierte Benzimidazole, Fischerbase-Derivate, Diphenylmalonsäuredinitrile, Oxalylamide, Campherderivate, Diphenylacrylate, Paraaminobenzoesäure (PABA) und deren Derivate, Salicylate, Benzophenone und noch weitere als UV-Filter bekannte Stoffklassen.

Erfindungsgemäss verwendbare, bevorzugte Triazinderivate entsprechen der Formel 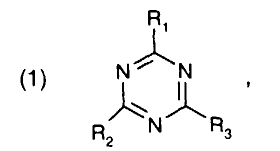 worin
- R₁, R₂ und R₃,: unabhängig voneinander Wasserstoff; OH; C₁-C₁₈-Alkoxy; -NH₂; -NH-R₄; -N(R₄)₂; -OR₄,
- R₄: C₁-C₅-Alkyl; nicht substituiertes oder durch einen, zwei oder drei OH-Gruppen, Carboxy, -CO-NH₂, C₁-C₅-Alkyl oder C₁-C₅-Alkoxy substituiertes Phenyl, Phenoxy, Anilino oder Pyrrolo, eine Methylidencampher-Gruppe; eine Gruppe der Formel -(CH=CH)ₘC(=O)-OR₄; eine Gruppe der Formel 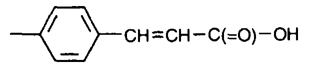 oder die entsprechenden Alkalimetall-, Ammonium-, Mono-, Di- oder Tri-C₁-C₄-Alkylammonium-, Mono-, Di- oder Tri-C₂-C₄-Alkanolammonium- Salze, oder deren C₁-C₃-Alkylester; oder einen Rest der Formel 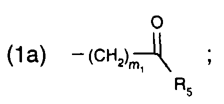
- R₅: Wasserstoff; nicht substituiertes oder durch eine oder mehrere OH-Gruppen substituiertes C₁-C₅-Alkyl; C₁-C₅-Alkoxy; Amino; Mono- oder Di-C₁-C₅-Alkylamino; M; einen Rest der Formel 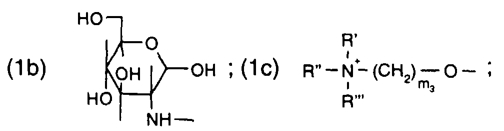 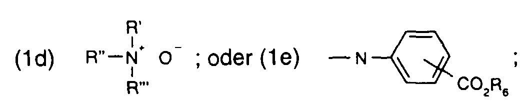 worin
- R', R" und R"': unabhängig voneinander nicht substituiertes oder durch eine oder mehrere OH-Gruppen substituiertes C₁-C₁₄-Alkyl;
- R₆: Wasserstoff; M; C₁-C₅-Alkyl; oder einen Rest der Formel -(CH₂)_{m₂}-O-T₁ ;
- M: ein Metallkation;
- T₁: Wasserstoff; oder C₁-C₈-Alkyl;
- m: 0 oder 1
- m₂: 1 bis 4; und
- m₃: 2 bis 14;
bedeuten.

Weitere bevorzugte, erfindungsgemäss verwendbare Triazinderivate entsprechen der Formel 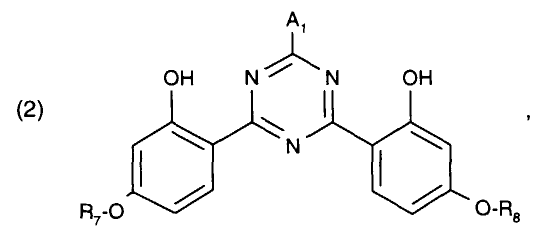 worin
- R₇ und R₈,: unabhängig voneinander, C₁-C₁₈-Alkyl; C₂-C₁₈-Alkenyl; einen Rest der Formel -CH₂-CH(-OH)-CH₂-O-T₁ ; oder
- R₇ und R₈: einen Rest der Formel 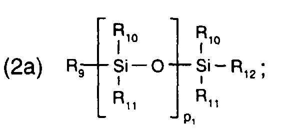
- R₉: die direkte Bindung; einen geradkettigen oder verzweigten C₁-C₄-Alkylenrest oder einen Rest der Formel 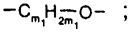
- R₁₀, R₁₁ und R₁₂,: unabhängig voneinander C₁-C₁₈-Alkyl; C₁-C₁₈-Alkoxy oder einen Rest der Formel 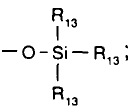
- R₁₃: C₁-C₅-Alkyl;
- m₁: 1 bis 4;
- p₁: 0 bis 5;
- A₁: einen Rest der Formel 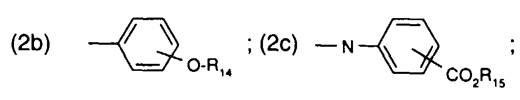 oder der Formel 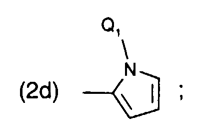
- R₁₄: Wasserstoff; C₁-C₁₀-Alkyl, -(CH₂CHR₁₆-O)_{n₁}-R₁₅ ; oder einen Rest der Formel -CH₂-CH(-OH)-CH₂-O-T₁;
- R₁₅: Wasserstoff; M; C₁-C₅-Alkyl; oder einen Rest der Formel -(CH₂)_{m₂}-O-(CH₂)_{m₃}-T₁ ;
- R₁₆: Wasserstoff; oder Methyl;
- T₁: Wasserstoff; oder C₁-C₈-Alkyl;
- Q₁: C₁-C₁₈-Alkyl;
- M: ein Metallkation;
- m₂ und m₃: unabhängig voneinander 1 bis 4; und
- n₁: 1 bis 16;
bedeuten.

Ganz besonders bevorzugte Triazinderivate der Formel (2) entsprechen den Formeln 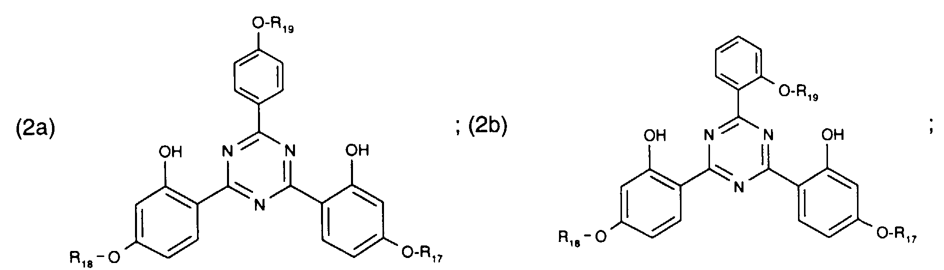 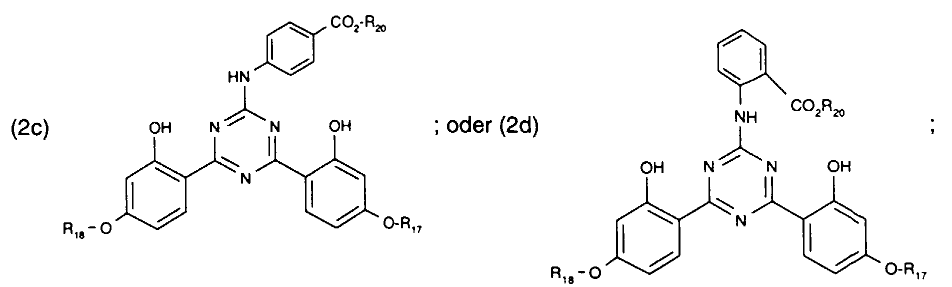 worin
- R₁₇ und R₁₈,: unabhängig voneinander, C₃-C₁₈-Alkyl; oder -CH₂-CH(-OH)-CH₂-O-T₁;
- R₁₉: C₁-C₁₀-Alkyl oder einen Rest der Formel 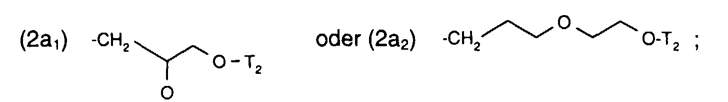
- R₂₀: Wasserstoff; M; C₁-C₅-Alkyl; -NH-C₁-C₅-Alkyl; vorzugsweise -NH-tert.alkyl; oder einen Rest der Formel -(CH₂)ₘ-O-T₂;
- T₁ und T₂: unabhängig voneinander, Wasserstoff; oder C₁-C₅-Alkyl; und
- m: 1 bis 4;
bedeuten.

Von ganz besonderem Interesse sind Verbindungen der Formel (2a) und (2b), worin
- R₁₇ und R₁₈,: unabhängig voneinander, C₁-C₁₈-Alkyl; oder -CH₂-CH(-OH)-CH₂-O-T₁;
- R₁₉: C₁-C₁₀-Alkyl;
und Verbindungen der Formel (2c) und (2d), worin
- R₁₇ und R₁₈,: unabhängig voneinander, C₁-C₁₈-Alkyl oder -CH₂-CH(-OH)-CH₂-O-T₁; und
- T₁: Wasserstoff; oder C₁-C₅-Alkyl;
bedeuten.

Im Vordergrund des Interesses stehen Triazinverbindungen der Formel (2a) - (2d), worin R₁₇ und R₁₈ dieselbe Bedeutung haben.

Weitere interessante erfindungsgemäss verwendbare Triazinverbindungen entsprechen der Formel 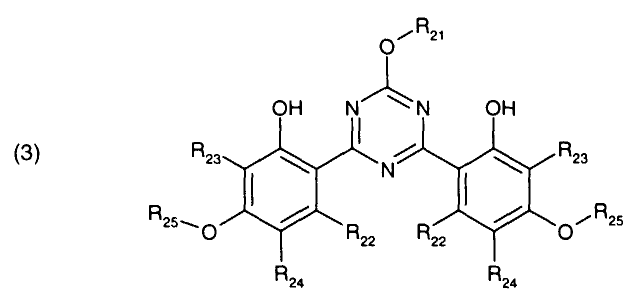 worin
- R₂₁: C₁-C₃₀-Alkyl; C₂-C₃₀-Alkenyl; nicht substituiertes oder durch C₁-C₅Alkyl mono- oder polysubstituiertes C₅-C₁₂-Cycloalkyl, C₁-C₅-Alkoxy-C₁-C₁₂-Alkyl; Amino-C₁-C₁₂-Alkyl; C₁-C₅-Monoalkylamino-C₁-C₁₂-Alkyl; C₁-C₅-Dialkylamino-C₁-C₁₂-Alkyl; einen Rest der Formel 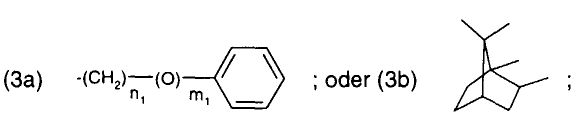 worin
- R₂₂, R₂₃ und R₂₄,: unabhängig voneinander, Wasserstoff, -OH; C₁-C₃₀-Alkyl, C₂-C₃₀-Alkenyl,
- R₂₅: Wasserstoff; oder C₁-C₅-Alkyl;
- m₁: 0 oder 1; und
- n₁: 1 bis 5;
bedeuten.

Bevorzugte Verbindungen entsprechen der Formel 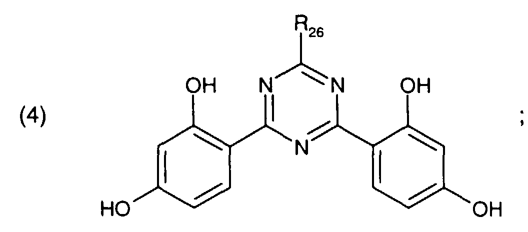 worin
R₂₆ 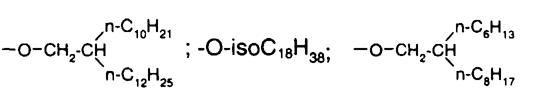 -O-n-C₁₈H₃₇; oder
-O-2-ethylhexyl; -O-(CH₂)₃-N(C₂H₅)₂; 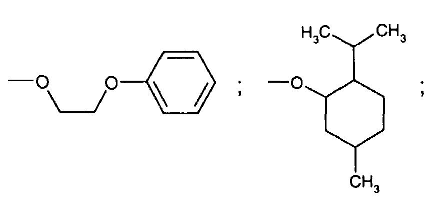 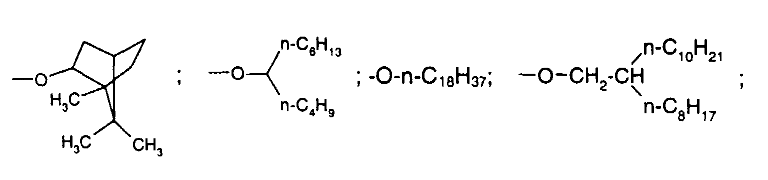 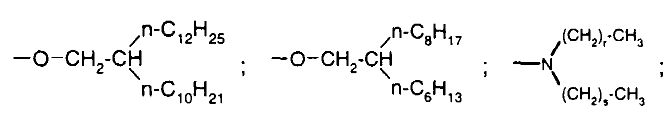 und
r und s unabhängig voneinander
0 bis 20;
bedeuten.

Beispielhafte, erfindungsgemäss verwendbare Triazinderivate entsprechen den Formeln 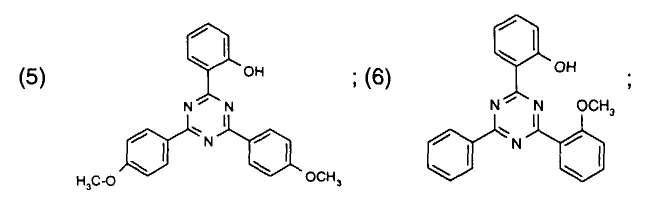 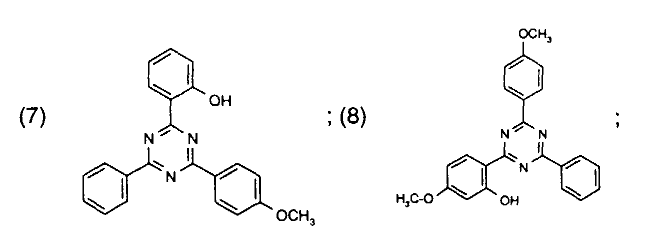 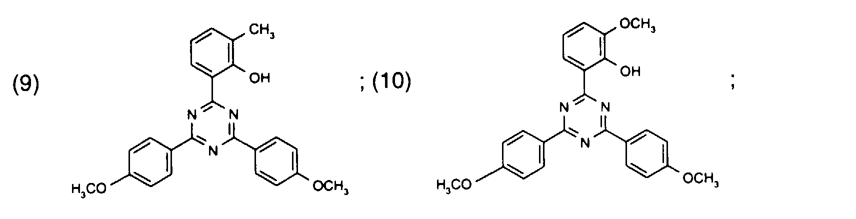 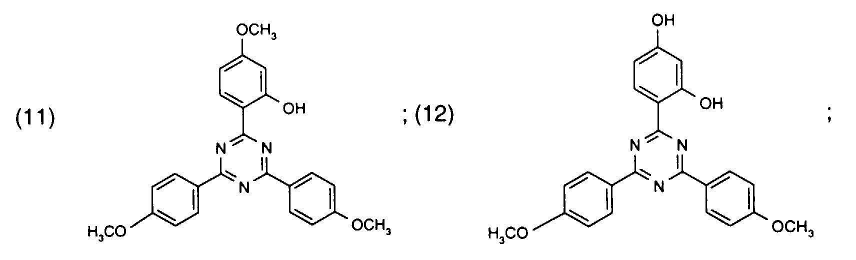 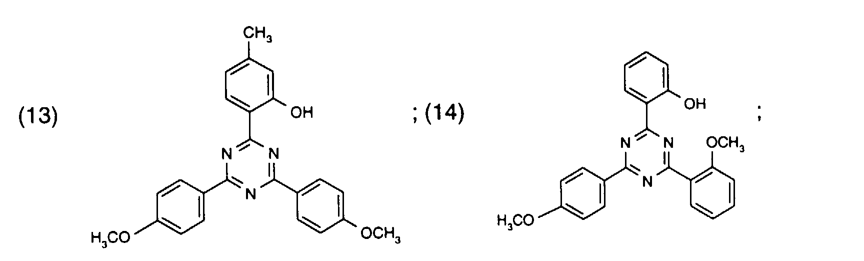 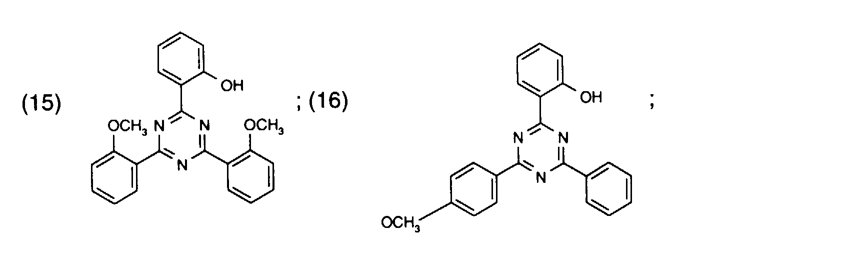 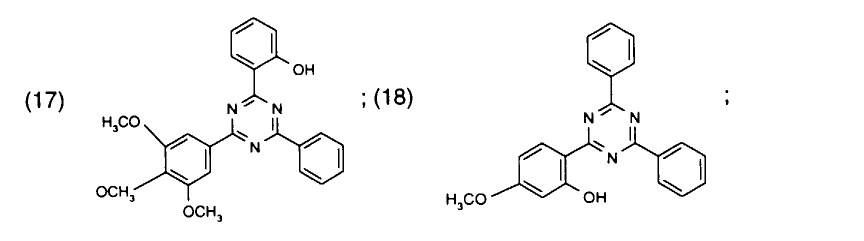 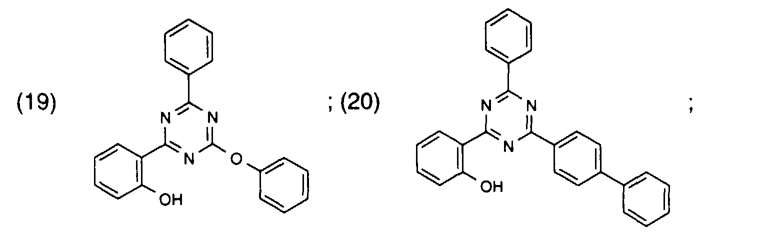 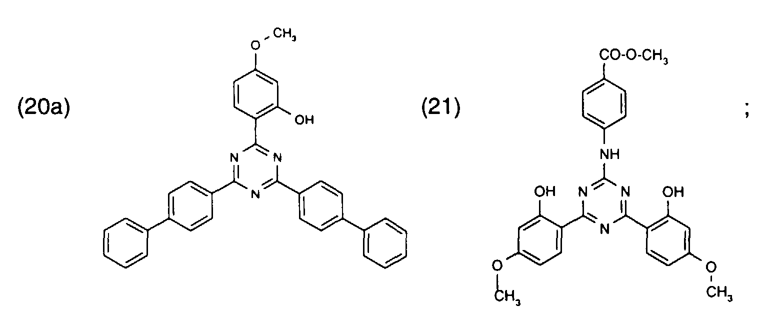 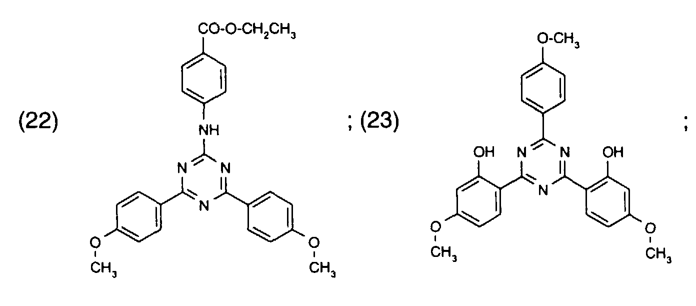 und 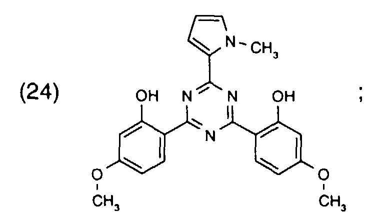 weiterhin 2,4,6-Tris(diisobutyl-4'-aminobenzalmalonat)-s-triazin und 2,4-Bis(diisobutyl-4-aminobenzalmalonat)-6-(4'-aminobenzylidencampher)-s-triazin.

Ebenfalls bevorzugte erfindungsgemäss verwendbare Triazinverbindungen sind in der EP-A-654469 beschrieben, wie z.B. die Verbindung der Formel

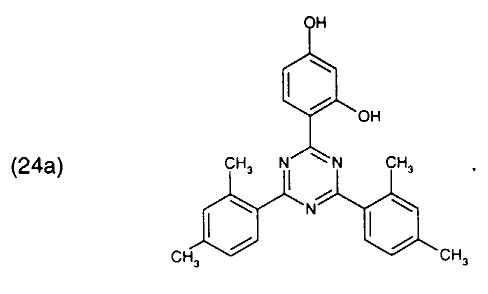

Erfindungsgemäss eignen sich insbesondere Triazinverbindungen, die z.B. in der EP-A-0,818450 beschrieben sind, wie z.B. die Verbindung der Formel

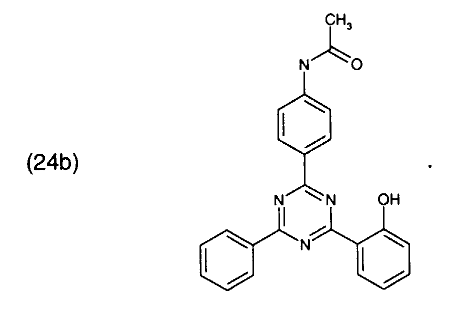

Ganz besonders bevorzugte erfindungsgemäss verwendbare Triazinderivate entsprechen der Formel 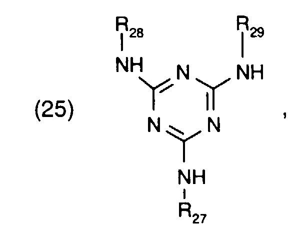 worin
- R₂₇, R₂₈ und R₂₉: unabhängig voneinander einen Rest der Formel 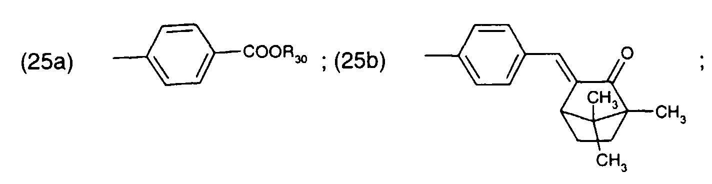 oder 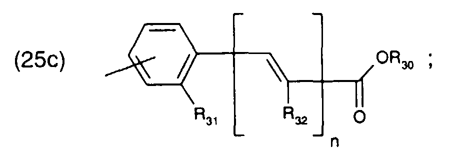
- R₃₀: Wasserstoff; Alkali-Metall; eine Ammoniumgruppe -N(R₃₃)₄,
- R₃₃: Wasserstoff; C₁-C₅-Alkyl; oder einen Polyoxyethylenrest, der 1 bis 10 Ethylenoxideinheiten aufweist und die endständige OH-Gruppe mit einem C₁-C₅-Alkohol verethert sein kann;
- R₃₁: Wasserstoff; -OH; oder C₁-C₆-Alkoxy;
- R₃₂: Wasserstoff oder -COOR₃₀; und
- n: 0 oder 1;
bedeuten.

Wenn R₃₀ Alkalimetall bedeutet, ist dies insbesondere Kalium oder ganz besonders Natrium. (R₃₃)₄ bedeutet insbesondere ein Mono-, Di- oder Tri-C₁-C₄-Alkylammoniumsalz, ein Mono-, Di- oder Tri-C₂-C₄-Alkanolammoniumsalz oder dessen C₁-C₃-Alkylester.

Wenn R₃₃ eine C₁-C₃-Alkylgruppe bedeutet, ist dies insbesondere eine C₁-C₂-Alkylgruppe, insbesondere eine Methylgruppe, und wenn R₃₃ einen Polyoxyethylenrest bedeutet, enthält dieser insbesondere 2 bis 6 Ethylenoxideinheiten.

Bevorzugte erfindungsgemäss einsetzbare Benzotriazolverbindungen entsprechen der Formel 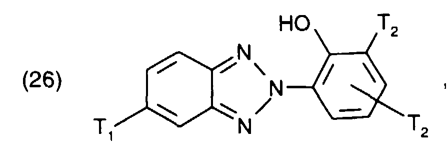 worin
- T₁: C₁-C₅-Alkyl oder vorzugsweise Wasserstoff; und
- T₂: C₁-C₅-Alkyl, vorzugsweise t-Butyl, oder phenylsubstituiertes C₁-C₄-Alkyl, insbesondere α,α-Dimethylbenzyl;
bedeuten.

Eine weitere bevorzugte Klasse erfindungsgemäss einsetzbarer Benzotriazolverbindungen entspricht der Formel 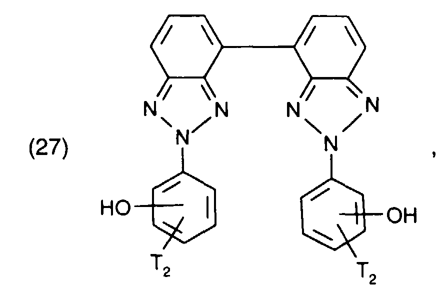 worin
T₂ die in Formel (26) angegebene Bedeutung hat.

Weitere, ganz besonders bevorzugte erfindungsgemäss einsetzbare Benzotriazolverbindungen entsprechen der Formel 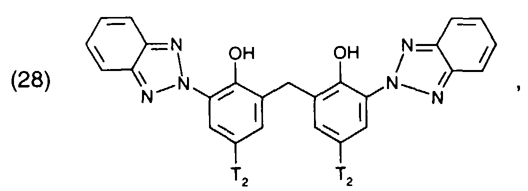 worin
- T₂: die in Formel (26) angegebene Bedeutung hat und vorzugsweise Methyl, t-Butyl oder iso-Octyl bedeutet.

Bevorzugte erfindungsgemäss einsetzbare, Vinylgruppen enthaltende Amide entsprechen der Formel

(29) R₃₃-(Y)ₘ-CO-C(R₃₄)=C(R₃₅)-N(R₃₆)(R₃₇),

worin
- R₃₃: C₁-C₅-Alkyl, vorzugsweise Methyl oder Ethyl, oder gegebenenfalls mit einer, zwei oder drei der Reste OH, C₁-C₅-Alkyl, C₁-C₅-Alkoxy oder CO-OR₃₃ substituiertes Phenyl;
- R₃₄, R₃₅, R₃₆ und R₃₇: unabhängig voneinander C₁-C₅-Alkyl, vorzugsweise Methyl oder Ethyl; oder Wasserstoff;
- Y: -NH oder -O-; bedeuten, und
- m: die oben angegebene Bedeutung hat

Bevorzugte Verbindungen der Formel (29) sind 4-Methyl-3-penten-2-on, Ethyl-3-methylamino-2-butenoat, 3-Methylamino-1-phenyl-2-buten-1-on und 3-Methylamino-1-phenyl-2-buten-1-on.

Bevorzugte erfindungsgemäss einsetzbare Zimtsäureamide entsprechen der Formel 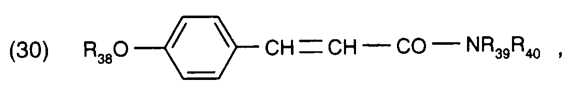 worin
- R₃₈: Wasserstoff oder C₁-C₅-Alkoxy, vorzugsweise Methoxy oder Ethoxy;
- R₃₉: Wasserstoff oder C₁-C₅-Alkyl, vorzugsweise Methyl oder Ethyl; und
- R₄₀: -(CONH)ₘ-Phenyl, worin m die oben angegebene Bedeutung hat und die Phenylgruppe gegebenenfalls durch einen, zwei oder drei der Reste OH, C₁-C₃-Alkyl, C₁-C₃-Alkoxy oder CO-OR₃₀ substituiert ist.

Vorzugsweise ist R₄₀ Phenyl, 4-Methoxyphenyl oder die Phenylaminocarbonyl-Gruppe.

Weitere bevorzugte Zimtsäurederivate sind 2-Ethylhexyl-4-methoxycinnamat oder -isoamylat oder u.a. die in den US-A-5 601 811 und WO 97/00851 offenbarten Zimtsäurederivate.

Bevorzugte erfindungsgemäss einsetzbare sulfonierte Benzimidazole entsprechen der Formel 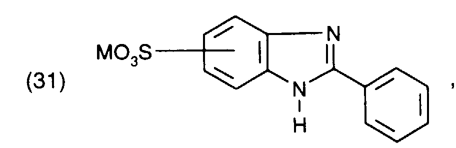 worin
- M: Wasserstoff oder ein Alkalimetall, vorzugsweise Natrium, ein Erdalkalimetall, wie z.B. Magnesium oder Calcium, oder Zink;
bedeutet.

Bevorzugte erfindungsgemäss verwendbare Fischer-Base-Aldehyde entsprechen der Formel 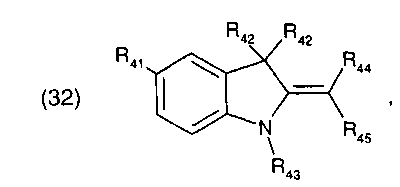 worin
- R₄₁: Wasserstoff; C₁-C₅-Alkyl; C₁-C₁₈-Alkoxy; oder Halogen;
- R₄₂: C₁-C₈-Alkyl; C₅-C₇-Cycloalkyl; oder C₆-C₁₀-Aryl;
- R₄₃: C₁-C₁₈-Alkyl oder ein Rest der Formel 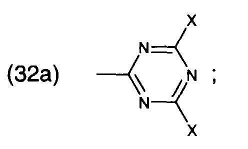
- R₄₄: Wasserstoff; oder ein Rest der Formel 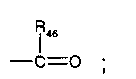
- R₄₅: 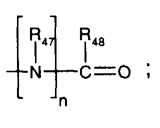 C₁-C₁₈-Alkoxy; oder einen Rest der Formel 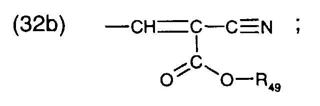
- R₄₆ und R₄₇: unabhängig voneinander Wasserstoff; oder C₁-C₅-Alkyl;
- R₄₈: Wasserstoff; C₁-C₅-Alkyl; C₅-C₇-Cycloalkyl; Phenyl; Phenyl-C₁-C₃-Alkyl;
- R₄₉: C₁-C₁₈-Alkyl;
- X: Hal; ein Rest der Formel 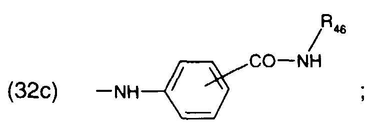 oder 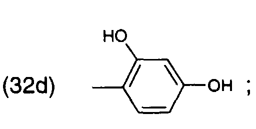 und
- n: 0 oder 1;
bedeuten.

Weitere bevorzugt einsetzbare Verbindungen entsprechen der Formel 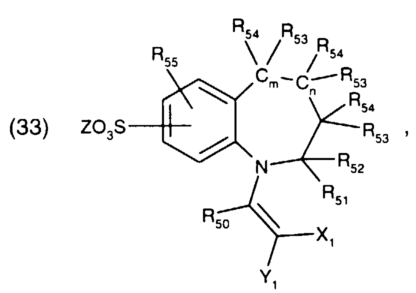 worin
- R₅₀, R₅₁, R₅₂, R₅₃, R₅₄: unabhängig voneinander Wasserstoff, C₁-C₈-Alkyl oder C₅-C₁₀-Cycloalkyl;
- R₅₅: Wasserstoff; C₁-C₈-Alkyl; C₅-C₁₀-Cycloalkyl; Hydroxy; C₁-C₈-Alkoxy; COOR₅₆; oder CONR₅₇R₅₈;
- R₅₆, R₅₇ und R₅₈: unabhängig voneinander Wasserstoff oder C₁-C₆-Alkyl;
- X und Y: unabhängig voneinander Wasserstoff, -CN; CO₂R₅₉; CONR₅₉R₆₀; oder COR₅₉;
wobei die Reste X und Y zusätzlich einen C₁-C₈-Alkylrest, ein C₅-C₁₀-Alkylrest, insbeondere Phenyl, oder einen Heteroarylrest mit 5 bis 6 Ringatomen sein kann, wobei ferner X und Y oder
- R₅₀: zusammen mit einem der Reste X und Y den Rest zur Vervollständigung eines 5- bis 7-gliedrigen Ringes bedeuten kann, der bis zu 3 Heteroatome, insbesondere Sauerstoff und/oder Stickstoff, enthalten kann, wobei die Ringatome insbesondere mit exocyclisch doppelt gebundenem Sauerstoff (Ketosauerstoff) und/oder C₁-C₈-Alkyl- und/oder C₅-C₁₀-Cycloalkylresten) substituiert sein können und/oder C=C-Doppelbindungen enthalten können;
- Z: Wasserstoff; Ammonium; Alkalimetallion; insbesondere Lithium, Natrium, Kalium, 1/2-Equivalente Erdalkalimetallion, vorzugsweise Calcium, Magnesium oder das Kation einer zur Neutralisation der freien Säuregruppe eingesetzte organische Stickstoffbase,
- R₅₉ und R₆₀: unabhängig voneinander Wasserstoff, C₁-C₈-Alkyl oder C₅-C₁₀-Cycloalkyl; und
- n und m: unabhängig voneinander 0 oder 1 bedeuten.

Bevorzugte erfindungsgemäss einsetzbare Diphenylmalonsäurenitrile entsprechen der Formel 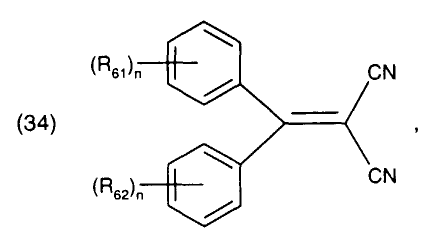 worin
- R₆₁ und R₆₂: unabhängig voneinander C₁-C₁₂-Alkyl; oder C₁-C₁₂-Alkoxy; und
- n: 0-3;
bedeuten.

Weitere erfindungsgemäss verwendbare organische UV-Filter entsprechen der Formel 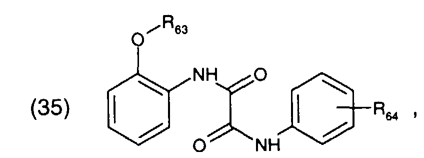 worin
R₆₃ und R₆₄ unabhängig voneinander C₁-C₅-Alkyl, insbesondere Ethyl;
bedeuten.

Weitere bevorzugte erfindungsgemäss einsetzbare chemische Verbindungsklassen von UV-Filtern:
- p-Aminobenzoesäurederivate (PABA), insbesondere 2-Ethylhexyl-4-dimethylaminobenzoat;
- Salicylsäurederivate, insbesondere 2-Ethylhexylsalicylate; Homosalate; und Isopropylsalicylate;
- Benzophenonderivate, insbesondere Benzophenon-2, -3, und -4;
- Dibenzoylmethanderivate, insbesondere 1-(4-tert-Butylphenyl)-3-(4-methoxyphenyl)propan-1,3-dion oder Butylmethoxydibenzoylmethan;
- Diphenylacrylate, insbesondere 2-Ethylhexyl-2-cyano-3,3-diphenylacrylat, Ethyl-2-cyano-3,3'-diphenylacrylat und 3-(Benzofuranyl)-2-cyanoacrylat;
- 3-lmidazol-4-yl-acrylsäure und 3-lmidazol-4-yl-acrylat;
- Benzofuranderivate, insbesondere die in den EP-A-582,189, US-A-5,338,539, und US-A-5-518,713 veröffentlichten p-Aminophenylbenzofuranderivate;
- Campherderivate, insbesondere 3-(4'-Methyl)benzylidenebornan-2-on, 3-Benzylidenbornan-2-on, N-[2(und 4)-2-Oxyborn-3-ylidenemethyl)benzyl]acrylamidpolymer, 3-(4'-Trimethylammonium)benzylidenbornan-2-on-methylsulfat, 3,3'-(1,4-Phenylenedimethin)-bis(7,7-dimethyl-2-oxobicyclo-[2.2.1]heptan-1-methansulfonsäure) und deren Salze, 3-(4'-sulfo)Benzylidenbornan-2-on und deren Salze; sowie
- Menthyl-o-aminobenzoat.

Die oben aufgeführten UV-Filter können erfindungsgemäss als Einzelverbindungen oder auch vorzugsweise als Gemische verwendet werden.

Vorzugsweise werden folgende Mischungen organischer UV-Filter verwendet:
- Mischungen aus Methylen-Bis-benzotriazolytetramethylbutylphenol und Octyltriazon;
- Mischungen aus Octyltriazon und Methylen-Bis-benzotriazolyltetramethylbutylphenol;
- Mischungen aus 2-[(2,4-methoxy)-phenyl]-4,6-bis-[(2-hydroxy-4-methoxy)-phenyl]-(1,3,5)-triazin und Methylen-bis-benzotriazolyltetramethylbutylphenol;
- Mischungen aus Methylen-bis-benzotriazolyltetramethylbutylphenol und Dioctylbutamidotriazon;
- Mischungen aus Methylen-bis-benzotriazolyltetramethylbutylphenol und Octyl-2,2'-Methylen-bis-[6-(2H-benztriazol-2-yl)-4-methyl-phenol,
- Mischungen aus Octyltriazon und Trisresorcinyltriazin;
- Mischungen aus 2,2'-Methylen-bis-[6-(2H-benztriazol-2-yl)-4-methyl-phenol, Octyltriazon und der Verbindung der Formel 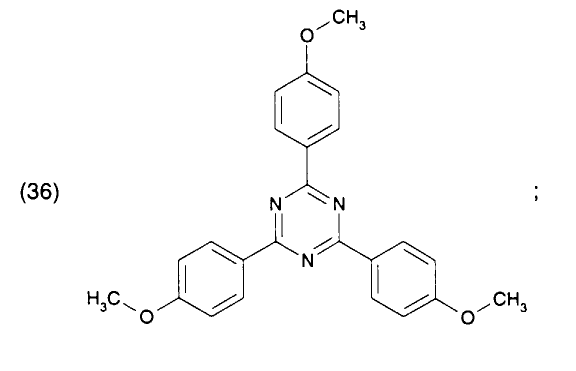
- Mischungen aus 2,2'-Methylen-bis-[6-(2H-benztriazol-2-yl)-4-methyl-phenol, Octyltriazon und der Verbindung der Formel 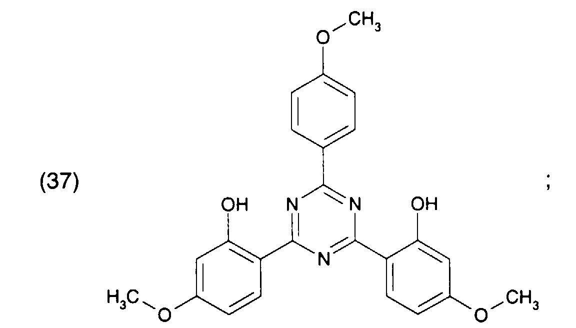
- Mischungen aus Methylen-bis-benzotriazolyltetramethylbutylphenol, Octyltriazon und der Verbindung der Formel 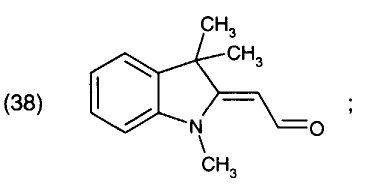
- Mischungen aus Methylen-bis-benzotriazolyltetramethylbutylphenol und der Verbindung der Formel 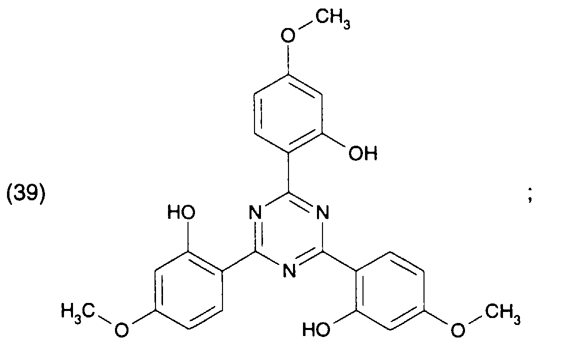
- Mischungen aus Methylen-bis-benzotriazolyltetramethylbutylphenol, Dioctylbutamidotriazon und der Verbindung der Formel (37).

In den oben definierten Resten sind C₁-C₁₈-Alkyl geradkettige oder verzweigte Alkylreste wie z.B. Methyl, Ethyl, n-Propyl, Isopropyl, n-Butyl, sek.Butyl, tert.Butyl, Amyl, Isoamyl oder tert.Amyl, Heptyl, Octyl, Isooctyl, Nonyl, Decyl, Undecyl, Dodecyl, Tetradecyl, Pentadecyl, Hexadecyl, Heptadecyl oder Octadecyl.

C₁-C₁₈-Alkoxyreste sind geradkettige oder verzweigte Alkylreste wie z.B. Methoxy, Ethoxy, n-Propoxy, Isopropoxy, n-Butoxy, sek.Butoxy, tert.Butoxy, Amyloxy, Isoamyloxy oder tert.Amyloxy, Heptyloxy, Octyloxy, Isooctyloxy, Nonyloxy, Decyloxy, Undecyloxy, Dodecyloxy, Tetradecyloxy, Pentadecyloxy, Hexadecyloxy, Heptadecyloxy oder Octadecyloxy.

C₂-C₁₈-Alkenyl bedeutet z.B. Allyl, Methallyl, Isopropenyl, 2-Butenyl, 3-Butenyl, Isobutenyl, n-Penta-2,4-dienyl, 3-Methyl-but-2-enyl, n-Oct-2-enyl, n-Dodec-2-enyl, iso-Dodecenyl, n-Dodec-2-enyl oder n-Octadec-4-enyl.

Die Herstellung der erfindungsgemäss verwendbaren Mischungen mikronisierter organischer UV-Filter kann auf verschiedene Weise erfolgen.

Einerseits können mindestens zwei der oben erwähnten organischen UV-Filter als Einzelsubstanzen beim Herstellungsprozess der Mikropartikel (Mikronisierung) gemischt werden.

Eine weitere Herstellungsmöglichkeit besteht darin, dass die bereits mikronisierten Einzelsubstanzen der UV-Filter innig miteinander gemischt werden.

Eine dritte Möglichkeit der Herstellung besteht darin, dass man mindestens zwei der oben erwähnten UV-Filter zusammenschmilzt. Nach Abkühlen der Schmelze entsteht ein homogener Composite, der auf übliche Art und Weise mikronisiert wird.

Die homogenen Composites aus mindestens zwei organischen UV-Filtern bilden einen weiteren Erfindungsgegenstand.

Einen weiteren Erfindungsgegenstand bilden Composites, die durch Einschmelzen von einem oder mehreren anorganischen Mikropigmenten in einen oder mehrere organische UV-Filter erhältlich sind.

Beispielhafte Mikropigmente sind z.B. TiO₂, ZnO, Eisenoxide oder andere anorganische Oxide, Glimmer (Mica) oder andere geeignete anorganische Mineralien, ferner auch Ti-, Erdalkali- oder Zinksalze von organischen Säuren.

Dadurch können gleichzeitig die unerwünschten photokatalytischen Eigenschaften einiger dieser anorganischen Mikropigmente (TiO₂, ZnO) unterdrückt werden und ihre positiven Eigenschaften zusätzlich voll genutzt werden.

Vorteilhafterweise werden die oben genannten anorganischen UV-Filter in Methylen-bis-benzotriazolyltetramethylbutylphenol eingeschmolzen. Der so entstandene Composite wird anschliessend auf übliche Weise mikronisiert.

Einen weiteren Erfindungsgegenstand bilden Composites, die durch Schmelzen von mindestens zwei elektrisch neutralen organischen UV-Filtern mit kationisch oder anionisch geladenen Verbindungen erhältlich sind.

Dazu werden kationisch oder anionisch geladene Verbindungen mit den entsprechenden organischen, elektrisch neutralen UV-Filtern geschmolzen und anschliessend abgekühlt. Durch dieses Verfahren lassen sich im anschliessenden Mikronisierungsschritt organische UV-Filterpigmente mit einer Permanentausrüstung aus einer positiven bzw. negativen Ladung herstellen. Eine solche Ausrüstung verhindert wirkungsvoll die Aggregation der mikronisierten Teilchen in den Sonnenschutzpräparaten, die bei einem Teilchendurchmesser von < 1µm auftreten kann. Eine sonst übliche "Coatung" dieser Teilchen mit abstossender Wirkung wird dann zum Teil überflüssig.

Als kationisch oder anionisch geladene Verbindungen können UV-Filter oder auch andere Verbindungen verwendet werden, die eine oder mehrere kationische oder anionische Gruppierungen aufweisen, wie z.B.
- N,N,N-Trimethyl-4-(2-oxoborn-3-ylidenmethyl)-anilinmethylsulfat;
- Campherbenzalkoniummethosulfat;
- Fettamine;
- Betaine, wie z.B. Cocoamidopropylbetain;
- Quats, wie z.B. Ricinolamidopropyltrimoniummethosulphat, Quarternium 18 , oder Cetyltrimethylammoniumbromid;
- Behensäure und andere organische Säuren, wie z.B. Isostearinsäure, Zitronensäuremonoglycerid oder Natriummethylcocoyltaurat;
- Phospholipide, wie z.B. Phosphatidylcholin, Phosphatidylserin oder Alkylaminoxid;
- Ceramide und Pseudoceramide und Phytosterole.

Die letztgenannten Verbindungen verleihen den mikronisierten UV-Filtern eine oleophobe Ausrüstung.

Der Anteil der kationischen oder anionischen Verbindungen im Composite liegt zwischen 0,001 und 5, vorzugsweise 0,01 bis 3 Gew.-%, bezogen auf das Gewicht des oder der UV-Filter.

Einen weiteren Erfindungsgegenstand bilden Composites, die durch Schmelzen von mindestens einem schwerlöslichen oder unlöslichen organischen UV-Filter mit Antioxidantien erhältlich sind.

Dazu werden der/die schwerlösliche(n) oder unlösliche(n) organische(n) UV-Filter mit Antioxidantien zusammengeschmolzen, abgekühlt und anschliessend auf übliche Weise mikronisiert.

Als erfindungsgemäss verwendbare Antioxidantien kommen alle organischen Substanzen mit Scavenger-Eigenschaften in Betracht, die sich zusammen mit organischen UV-Filtern schmelzen lassen. Man erhält neuartige Mikropigmente, die gleichzeitig die Bräunung der Haut verhindern und antioxidative Wirkung auf ihrer Oberfläche bieten. Diese Eigenschaft ist beim kosmetischen Sonnenschutz erwünscht, da unter UV- und Lichteinfluss sowohl in Formulierungen als auch auf der Haut schädliche Radikale gebildet werden können. Diese können z.B. zur sogenannten Mallorca-Akne oder zu vorzeitiger Hautalterung führen. Durch die Ausrüstung der mikronisierten UV-Filter mit Antioxidantien wird neben dem Schutz vor UV-Schäden und der Verhinderung der Bräunung gleichzeitig ein Schutz gegen den photochemischen Abbau von Bestandteilen in der Sonnenschutzformulierung erreicht.

Der Anteil der Antioxidantien im Composite liegt dabei gewöhnlich zwischen 0,001 und 30, vorzugsweise 0,01 bis 3 Gew.-%, bezogen auf das Gewicht des/der UV-Filter.

Besonders vorteilhaft ist ein Anteil von Antioxidantien in Mikropigmenten, wenn diese neben organischen UV-Filtern die oben erwähnten photokatalytisch aktiven anorganischen Mikropigmente, wie z.B. Titandioxid, Zinkoxid (auch gecoatet) oder andere geeignete anorganischen Oxide, wie z.B. Eisenoxid enthalten.

Als beispielhafte Antioxidantien seien die folgenden Verbindungen aufgeführt:
- Tocopherole, wie z.B. α-Tocopherol (CAS 59-02-9), Tocopherylacetat, Vitamin E Succinat,
- Ellagsäure 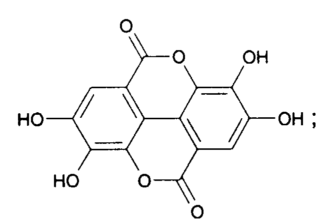
- Propylgallat (CAS 121-79-9) 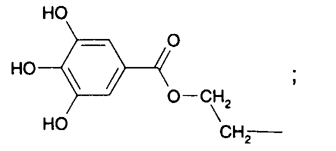
- N-butyliertes Hydroxytoluol (BHT; CAS 128-37-0);
- butyliertes Hydroxyanisol (BHA);
- 2,4,6-Tris(3,5-di-t-butyl-4-hydroxybenzyl)mesitylen (CAS 1709-70-2) 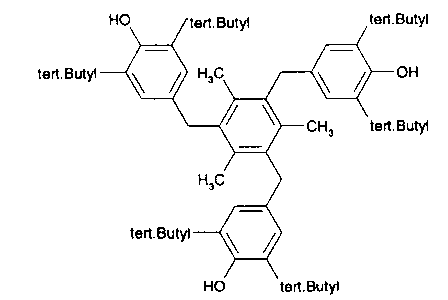
- Tetrakis-[methylen-3(3',5'-di-t-butyl-4'-hydroxyphenyl)propionat]methan (CAS 6683-19-8);
- Verbindung der Formel 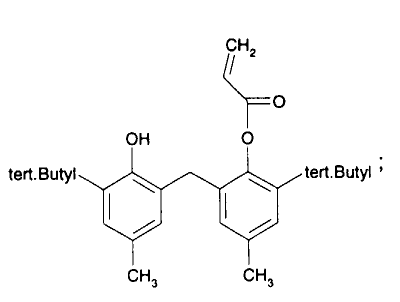
- Verbindung der Formel 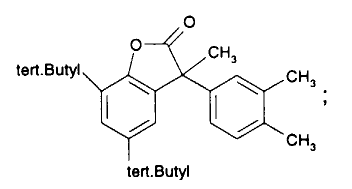
- Vanillin;
- Ubichinon;
- Ferulasäure und -Derivate;
- Rutinsäure und -Derivate;
- Urocaninsäure und -Derivate; und
- Propolis.

Vorzugsweise werden folgende Mischungen aus Antioxidantien und organischen UV-Filtern verwendet:
- Mischungen aus Methylen-bis-benzotriazolyltetramethylbutylphenol, Octyltriazon, Titandioxid und Tocopherol,
- Mischungen aus 2,2'-Methylen-bis-[6-(2H-benztriazol-2-yl)-4-methyl-phenol, Octyltriazon, Trisresorcinyltriazin und Vitamin E
- Mischungen aus Methylen-bis-benzotriazolyltetramethylbutylphenol, Octyltriazon , Verbindung der Formel 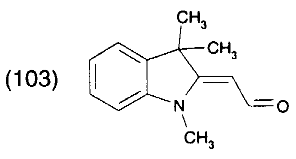 ("Fischeraldehyd") und Verbindung der Formel

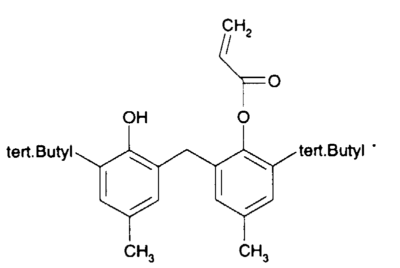

Einen weiteren Erfindungsgegenstand bilden Composites, die durch Einschmelzen von schmelzbaren kosmetischen, pflanzlichen und pharmazeutischen Wirkstoffen in organische UV-Filter erhältlich sind.

Generell können mikronisierte UV-Filter als Träger von hochaktiven Stoffen, insbesondere kosmetischen und/oder pharmazeutischen Wirksubstanzen genutzt werden. Der Vorteil derartiger Composites liegt in der Möglichkeit, den bzw. die aktiven Stoffe aus dem Festkörper freizusetzen (slow release). Eine langsame Freisetzung garantiert über die gesamte Nutzungsdauer der UV-Pigmente auch die gleichmässige Wirksamkeit von hochaktiven Wirkstoffen, wie z.B. Entzündungshemmern, Pflegewirkstoffen oder Spurenelementen, wie z.B. Zn²⁺ oder Mg²⁺.

Als beispielhaft einsetzbare Wirkstoffe seien erwähnt:
- Wirkstoffe zur antimikrobiellen Ausrüstung und gleichzeitigen antiinflamatorischen Wirkung, wie z.B. Triclosan oder Diclosan;
- entzündungshemmende Wirkstoffe, wie z.B. Farnesol, Panthenol oder Avocadoöl;
- Wirkstoffe mit Deo- bzw. Antiperspirantwirkung, wie z.B. Zn-Ricinoleate und Alkylcitrate,
- Undecylensäure und deren Derivate (z.B. Diethanolamide)
- Zinkundecylat;
- Pyrithione, wie z.B. Natriumpyrithion;
- eingeschmolzene Riechstoffe oder Riechstoffgemische, wie z.B. Menthol, Geraniol usw., die diesen Mikropigmenten und den Formulierungen, die diese enthalten, einen permanenten und gleichintensiven Geruch verleihen.

Zur Herstellung der mikronisierten organischen UV-Filter bzw. der Mikropigmentgemische können alle bekannten Verfahren, die für die Herstellung von Mikropartikeln geeignet sind, genutzt werden, wie z.B.:
- Nassmahlung mit einem hartem Mahlkörper wie z.B. Zirkoniumsilikat und einem Schutztensid oder einem Schutzpolymeren in Wasser oder einem geeigneten organischen Lösungsmittel;
- Sprühtrocknung aus einem geeigneten Lösungsmittel, wie z.B. wässrige oder organische Lösungsmittel enthaltende Suspensionen oder echte Lösungen in Wasser, Ethanol, Dichlorethan, Toluol, N-Methylpyrrolidon u.a..
- Durch Entspannung von superkritischen Flüssigkeiten (z.B. CO₂) nach dem RESS-Prozess (Rapid Expansion of Supercritical Solutions), in denen der oder die UV-Filter gelöst ist/sind oder Entspannung von flüssigem Kohlendioxid gemeinsam mit einer Lösung eines oder mehrerer UV-Filter in einem geeigneten organischen Lösungsmittel;
- durch Umfällen aus geeigneten Lösungsmitteln, einschliesslich superkritischen Flüssigkeiten (GASR-Prozeß = Gas Anti-Solvent Recrystallisation / PCA-Prozess = Precipitation with Compressed Antisolvents).

Als Mahlapparate zur Herstellung der erfindungsgemässen mikronisierten organischen UV-Absorber können z.B. eine Düsen-, Kugel-, Vibrations- oder Hammermühle, vorzugsweise eine Hochgeschwindigkeits-Rührmühle verwendet werden. Die Mahlung erfolgt vorzugsweise mit einer Mahlhilfe, wie z.B. einem alkylierten Vinylpyrrolidon-Polymer, einem Vinylpyrrolidon-Vinylacetat-Copolymer, einem Acylglutamat, einem Alkylpolyglucosid, Ceteareth-25 oder insbesondere einem Phospholipid.

Die so erhaltenen Mikropigmente bzw. Gemische aus Mikropigmenten haben gewöhnlich eine mittlere Partikelgrösse von 0,02 bis 2, vorzugsweise 0,05 bis 1,5, und ganz besonders von 0,1 bis 1,0 nm.

Auf Grund ihrer Lipophilität lassen sie sich alleine oder zusammen mit anderen löslichen organischen UV-Absorbern nach bekannten Methoden gut in öl- und fetthaltige kosmetische Formulierungen, wie z.B. Öle, O/W- oder W/O-Emulsionen, Fettstifte oder Gele einar-beiten.

Überraschend erhält man Formulierungen mit gleicher oder verbesserter Schutzwirkung bei Verwendung von weniger oder gar keinen löslichen UV-Absorbem.

Einen weiteren Erfindungsgegenstand bildet ein kosmetische Formulierung, enthaltend ein Gemisch aus Mikropigmenten, gegebenenfalls einen oder mehrere Antioxidantien und/oder anorganische Pigmente und/oder eine kationische bzw. anionische Verbindung, sowie kosmetisch verträgliche Träger- oder Hilfsstoffe.

Erfindungsgemässe kosmetische Formulierungen beinhalten verschiedene kosmetische Mittel. Insbesondere kommen z.B. die folgenden Mittel in Betracht:
- Mittel zur Hautpflege, wie z.B. Hautwasch- und Reinigungsmittel in Form von stückförmigen oder flüssigen Seifen, Syndets oder Waschpasten,
- Badepräparate, wie z.B. flüssige (Schaumbäder, Milche, Duschpräparate) oder feste Badepräparate, wie z.B. Badetabletten und Badesalze;
- Hautpflegemittel, wie z.B. Hautemulsionen, Mehrfachemulsionen oder Hautöle;
- Dekorative Körperpflegemittel, wie z.B. Gesichts-Make-ups in Form von Tages- oder Pudercremes, Gesichtspuder (lose und gepresst), Rouge oder Creme-Make-ups, Augenpflegemittel, wie z.B. Lidschattenpräparate, Wimperntusche, Eyeliner, Augencremes oder Eye-Fix-Cremes; Lippenpflegemittel, wie z.B. Lippenstift, Lip Gloss, Lippenkonturstift, Nagelpflegemittel, wie Nagellack, Nagellackentferner, Nagelhärter, oder Nagelhautentferner;
- Intimpflegemittel, wie z.B. Intim-Waschlotionen oder Intimsprays;
- Fusspflegemittel, wie z.B. Fussbäder, Fusspuder, Fusscremes bzw. Fussbalsame, spezielle Deomittel und Antitranspirantien oder hornhautbeseitigende Mittel;
- Lichtschutzmittel, wie Sonnenmilche, -lotionen, -cremes, -öle, Sun-blockers oder Tropicals, Vorbräunungspräparate oder After-sun-Präparate;
- Hautbräunungsmittel, wie z.B. Selbstbräunungscremes;
- Depigmentierungsmittel, wie z.B. Präparate zur Hautbleichung oder Mittel zur Hautaufhellung;
- Insektenabweisende Mittel ("Repellents"), wie z.B. Insektenöle, -lotionen, -sprays, oder -stifte;
- Deodorantien, wie Deosprays, Pumpsprays, Deogele, -stifte oder -roller;
- Antitranspirantien, wie z.B. Antitranspirantstifte, -cremes oder -roller;
- Mittel zur Reinigung und Pflege von unreiner Haut, wie z.B. Syndets (fest oder flüssig), Peeling- oder Scrubb-Präparate oder Peeling-Masken;
- Haarentfernungsmittel in chemischer Form (Depilation), wie z.B. Haarentfernungspulver, flüssige Enthaarungsmittel, cremige oder pastöse Enthaarungsmittel, Enthaarungsmittel in Gelform oder Aerosolschäume;
- Rasiermittel, wie z.B. Rasierseife, schäumende Rasiercremes, nichtschäumende Rasiercremes, Rasierschäume, -gele, Preshave-Präparate für die Trockenrasur, Aftershaves oder Aftershave-Lotionen;
- Duftmittel, wie z.B. Duftwässer (Eau de Cologne, Eau de Toilette, Eau de Parfum, Parfum de Toilette, Parfüm), Parfümöle oder Parfümcremes;
- Mittel zur Zahn-, Zahnersatz- und Mundpflege, wie z.B. Zahncremes, Gel-Zahncremes, Zahnpulver, Mundwasserkonzentrate, Anti-Plaque-Mundspülungen, Prothesenreiniger oder Prothesenhaftmittel;
- Kosmetische Mittel zur Haarbehandlung, wie z.B. Haarwaschmittel in Form von Schampoos, Haarkonditioniermittel, Haarpflegemittel, wie z.B. Vorbehandlungsmittel, Haarwasser, Frisiercremes, Frisiergele, Pomaden, Haarspülungen, Kurpackungen, Intensivhaarkuren, Mittel zur Haarverformung, wie z.B. Wellmittel zur Herstellung von Dauerwellen (Heisswelle, Mildwelle, Kaltwelle), Haarglättungspräparate, flüssige Haarfestiger, Haarschäume, Haarsprays, Blondiermittel, wie. z.B. Wasserstoffperoxidlösungen, aufhellende Schampoos, Blondiercremes, Blondierpulver, Blondierbreie oder -öle, temporäre, semitemporäre oder permanente Haarfärbemittel, Präparate mit selbstoxidierenden Farbstoffen, oder natürliche Haarfärbemittel, wie Henna oder Kamille.

Diese aufgezählten Endformulierungen können in verschiedenen Darreichungsformen vorliegen, wie z.B.
- in Form von flüssige Zubereitungen als einer W/O- O/W-, O/W/O-, W/O/W-, PIT- und aller Arten von Mikroemulsionen
- in Form eines Gels,
- in Form eines Öls, einer Creme, Milch oder Lotion,
- in Form eines Pulvers, eines Lacks, einer Tablette oder Make-Ups,
- in Form eines Stiftes,
- in Form eines Sprays (Spray mit Treibgas oder Pumpspray) oder eines Aerosols,
- in Form eines Schaumes, oder
- in Form einer Paste.

Vorteilhaft können die erfindungsgemässen kosmetischen Formulierungen weitere Substanzen enthalten, die UV-Strahlung im UVB-Bereich absorbieren. Die Gesamtmenge der Filtersubstanzen ist dabei 0,1 bis 30, vorzugsweise 0,5 bis 10 Gew.-%, insbesondere 1 bis 6 Gew.-%, bezogen auf das Gesamtgewicht der Zusammensetzung.

Insbesondere kommen als zusätzliche UVB-Filter öllösliche, nicht mikronisierte Verbindungen in Betracht, wie z.B. organische UV-Absorber aus der Klasse der p-Aminobenzoesäurederivate, Salicylsäurederivate, Benzophenonderivate, Dibenzoylmethanderivate, Diphenylacrylatderivate, Benzofuranderivate, polymere UV-Absorber, enthaltend eine oder mehrere silizium-organische Reste, Zimtsäurederivate, Campherderivate, Trianilino-s-Triazinderivate, Phenylbenzimidazolsulfonsäure und deren Salze, Menthyl-Anthranilat, Benzotriazolderivate, und/oder ein anorganisches Mikropigment ausgewählt aus mit Aluminiumoxid oder Siliciumdioxid umhülltem TiO₂, Zinkoxid oder Mica.
- Beispielhafte Verbindungen für p-Aminobenzoesäurederivate: 4-Aminobenzoesäure (PABA); Ethyldihydroxypropyl-PABA der Formel 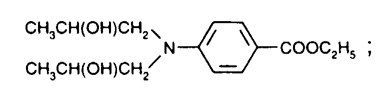 PEG-25-PABA der Formel 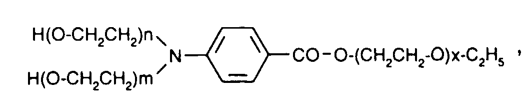 worin m, n und x dieselbe Bedeutung haben und je höchstens 25 bedeuten;
   Octyldimethyl PABA der Formel 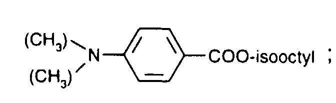 oder Glycylaminobenzoat der Formel 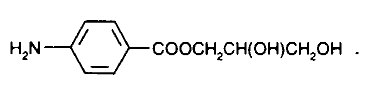
- Beispielhafte Verbindungen für Salicylsäurederivate:
   Homomenthylsalicylat der Formel 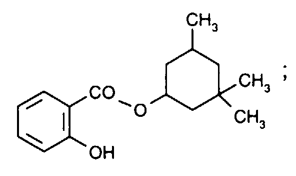 Triethanolaminsalicylat der Formel 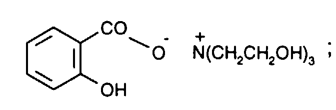 Amyl-p-dimethylaminobenzoat der Formel 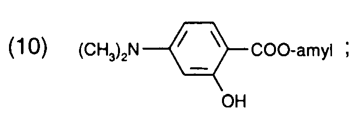 Octylsalicylat der Formel 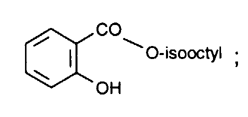 oder 4-lsopropylbenzylsalicylat der Formel 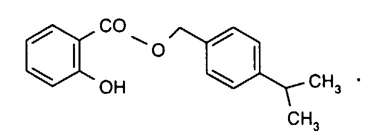
- Beispielhafte Verbindungen für Benzophenonderivate: Benzophenon-3-(2-hydroxy-4-methoxybenzophenon), Benzophenon-4-(2-hydroxy-4-methoxybenzophenon-5-sulfonsäure) oder Benzophenon-8-(2,2'-dihydroxy-4-methoxybenzophenon).
- Beispielhafte Verbindungen für Dibenzoylmethanderivate: Butylmethoxydibenzoylmethan-[1-(4-tert.-butyl)-3-(4-methoxyphenyl)propan-1,3-dion].
- Beispielhafte Verbindungen für Diphenylacrylatderivate: Octocrylen-(2-ethylhexyl-2-cyano-3,3'-diphenylacrylat) oder etocrylen-(ethyl-2-cyano-3,3'-diphenylacrylat).
- Beispielhafte Verbindungen für Benzofuranderivate: 3-(Benzofuranyl)-2-cyanoacrylat, 2-(2-Benzofuranyl)-5-tert.-butylbenzoxazol oder 2-(p-Aminophenyl)benzofuran und insbesondere die Verbindung der Formel 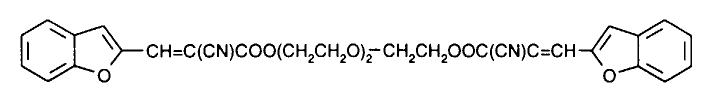 oder 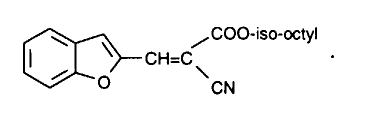
- Beispielhafte Verbindungen für polymere UV-Absorber, die eine oder mehrere silizium-organische Reste enthalten:
   Benzylidenmalonatderivat, insbesondere die Verbindung der Formel 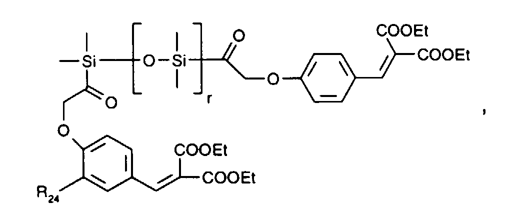 worin
   - R₂₄: Wasserstoff oder Methoxy und
   - r: ungefähr 7; die Verbindung der Formel 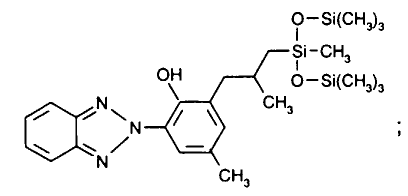 oder 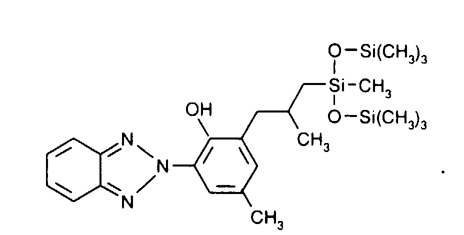
- Beispielhafte Verbindungen für Zimtsäureester:
   Octylmethoxycinnamat (4-Methoxyzimtsäure-2-ethylhexylester), Diethanolaminmethoxycinnamat (Diethanolaminsalz der 4-Methoxyzimtsäure), Isoamyl-p-methoxycinnamat (4-Ethoxyzimtsäure-2-isoamylester), 2,5-Diisopropylmethylcinnamat oder ein Zimtsäureamidoderivat.
- Beispielhafte Verbindungen für Campherderivate:
   4-Methyl-benzylidencampher [3-(4'-Methyl)benzyliden-bornan-2-on], 3-Benzylidencampher (3-Benzyliden-bornan-2-on), Polyacrylamidomethylbenzylidencampher {N-[2(und 4)-2-oxyborn-3-yliden-methyl)benzyl]acrylamidpolymer}, Trimonium-benzylidencamphersulfat-[3-(4'-trimethylammonium)-benzyliden-bornan-2-on-methylsulfat], Terephthalylidendicampher-Sulfonsäure {3,3'-(1,4-Phenylendimethin)-bis-(7,7-dimethyl-2-oxo-bicyclo-[2.2.1]heptan-1-methansulfonsäure} oder deren Salze, oder Benzylidencampher-Sulfonsäure [3-(4'-sulfo)benzylidenbornan-2-on] oder deren Salze.
- Beispielhafte Verbindungen für Trianilino-s-triazinderivate:
   Octyltriazin[2,4,6-trianilino-(p-carbo-2'-ethyl-1'-oxy)-1,3,5-triazin, sowie die in der US-A-5,332,568, US-A-5,252,323, WO 93/17002 und WO 97/03642 und EP-A-0,517,104 beschriebenen Trianilino-s-triazinderivate.
- Beispielhafte Verbindungen für Benzotriazole:
   2-(2-Hydroxy-5-methyl-phenyl)benzotriazol.

Die folgenden Beispiele dienen zur Erläuterung der Erfindung, ohne sie auf diese zu beschränken. Die kosmetischen Wirksubstanzen sind vorrangig mit ihrer INCl-Bezeichnung (INCl = International Nomenclature of Cosmetical Ingredients) angegeben.

### Beispiel 1

50 Teile Methylene Bis-benzotriazolyl Tetramethylbutylphenol und 50 Teile Octyl Triazone werden zusammen mit einem Mahlkörper aus Zirkoniumsilikat-Sand, einem Schutztensid (Alkyl Polyglucoside) und Wasser in einer Perl-Mühle zu einem Misch-Mikropigment mit einem d₅₀ von 190 nm vermahlen. Nach Abtrennen des Mahlkörpers kann die Suspension des Misch-Mikropigments zur Herstellung von Sonnenschutzformulierungen verwendet werden.

### Beispiel 2:

32 Teile Octyl Triazone, 1 Teil Cetyltrimethylammoniumbromid und 66 Teile Methylene Bis-benzotriazolyl Tetramethylbutylphenol werden homogen zusammengeschmolzen. Es wird rasch auf Raumtemperatur abgekühlt und die erstarrte Schmelze mechanisch (Schlagmühle) zerkleinert. Dieses so erhaltene Pulver wird in Wasser angeschlämmt, Decyl Glycoside zugefügt und zusammen mit einem Mahlhilfsmittel ('schwerer Sand') bis auf eine Teilchengröße von d₅₀ 200 nm Durchmesser mikronisiert. Nach Abtrennung des Mahlhilfsmittels erhält man eine wässrige Suspension des mikronisierten UV-Absorbercomposites. Diese Suspension wird mit Zitronensäure leicht sauer eingestellt und kann zur Herstellung von kosmetischen und pharmazeutischen Formulierungen verwendet werden.

### Beispiel 3:

25 Teile 2-[(2,4-methoxy)-phenyl]-4,6-bis-[(2-hydroxy-4-methoxy)-phenyl]-(1,3,5)-triazine, 74 Teile Methylene Bis-benzotriazolyl Tetramethylbutylphenol und 1 Teil Tetrakis[methylene-3(3',5'-di-t-butyl-4'-hydroxyphenyl)propionate]methane werden homogen zusammengeschmolzen. Es wird rasch auf Raumtemperatur abgekühlt und die erstarrte Schmelze mechanisch (Schlagmühle) zerkleinert. Dieses so erhaltene Pulver wird in Wasser angeschlämmt, erst Decyl Glycoside - nach Mahlfortschritt Ceteareth-25 zugefügt und zusammen mit einem Mahlhilfsmittel ('schwerer Sand') bis auf eine Teichengröße von d₅₀ 190 nm Durchmesser mikronisiert. Nach Abtrennung des Mahlhilfsmittels erhält man eine wässrige Suspension des mikronisierten UV-Absorbercomposites, die zur Herstellung von kosmetischen und pharmazeutischen Formulierungen verwendet werden kann.

### Beispiel 4:

In 75 Teilen geschmolzenem Methylene Bis-benzotriazolyl Tetramethylbutylphenol werden 25 Teile Dioctyl Butamido Triazone gelöst. Es wird rasch abgekühlt, das Gemisch mechanisch zu feinem Pulver zerkleinert und danach mit einem Mahlkörper aus Zirkoniumsilikat-Sand, einem Schutztensid (Phospholipid) und Wasser zu einem Mikropigment mit einem d₅₀ 300 nm vermahlen. Die vom Mahlkörper abgetrennte Suspension des Mikropigmentes wird zur Herstellung von Sonnenschutzformulierungen verwendet.

### Beispiel 5:

In 70 Teilen geschmolzenes Methylene Bis-benzotriazolyl Tetramethylbutylphenol werden mit 24 Teilen Octyl Triazone, 5 Teilen Titanium Dioxide und einem Teil Tocopherol vermischt. Es wird rasch abgekühlt, das Gemisch mechanisch zu feinem Pulver zerkleinert und danach mit einem Mahlkörper aus Zirkoniumsilikat-Sand, einem Schutztensid (Alkyl Polyglucosid) und Wasser zu einem Mikropigment vermahlen. Die vom Mahlkörper abgetrennte Suspension des Mikropigmentes wird zur Herstellung von Sonnenschutzformulierungen verwendet.

In den folgenden Beispielen 6 bis 11 werden analog zu den Beispielen 1 und 2 Suspensionen von Mikrocomposites folgender Zusammensetzungen hergestellt:

### Beispiel 6:

60 Teile 2,2'-Methylen-bis-[6-(2H-benzotriazol-2-yl)-4-methyl-phenol,
20 Teile Octyl triazone, 19 Teile Tris Resorcinyl Triazin und 1 Teil Vitamin E, eingestellt auf pH 6,5 mit Zitronensäure.

### Beispiel 7:

60 Teile 2,2'-Methylen-bis-[6-(2H-benztriazol-2-yl)-4-methyl-phenol,
20 Teile Octyl Triazone und 20 Teile der Verbindung der Formel

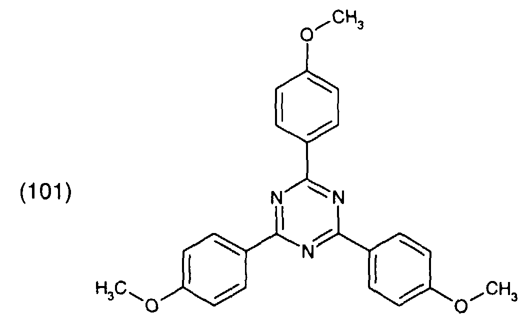

### Beispiel 8:

59 Teile 2,2'-Methylen-bis-[6-(2H-benztriazol-2-yl)-4-methyl-phenol,
20 Teile Octyl Triazone,
20 Teile der Verbindung der Formel 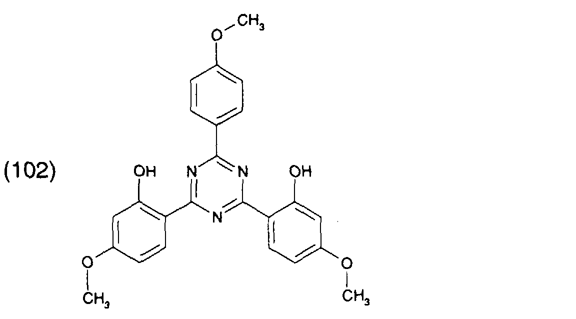
und eingestellt auf pH 6,5 mit Zitronensäure.

### Beispiel 9:

75 Teile Methylene Bis-benzotriazolyl Tetramethylbutylphenol,
10 Teile Octyl Triazone (Mahlung bei pH < 5, eingestellt mit Zitronensäure),
14 Teile der Verbindung der Formel 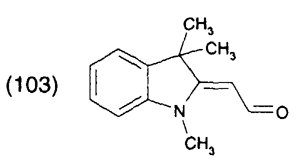 ("Fischeraldehyd") und
1 Teil der Verbindung der Formel

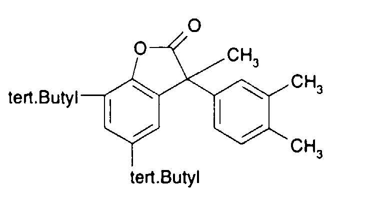

### Beispiel 10:

80 Teile Methylene Bis-benzotriazolyl Tetramethylbutylphenol, und
20 Teile der Verbindung der Formel

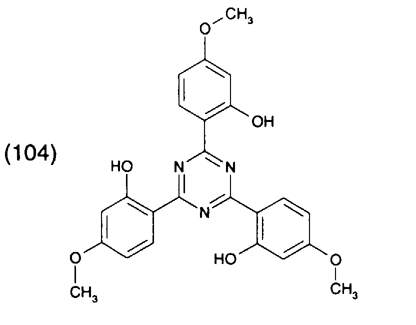

### Beispiel 11:

50 Teile Methylene Bis-benzotriazolyl Tetramethylbutylphenol,
10 Teile Dioctyl Butamido Triazone (Mahlung bei pH < 5, eingestellt auf pH 6,5 mit Zitronensäure) und
20 Teile der Verbindung der Formel (102).

### Beispiel 12: O/W-Lotion zur Bräunungsverhinderung

| | | % |
|---|---|---|
| A | Polyglyceryl-3 Methylglucose Distearate | 2,0 |
| | Decyl Oleate | 5,7 |
| | Isopropyl Palmitate | 6,0 |
| | Caprylic/Capric Triglycerid | 7,5 |
| | | |
| B | Glycerin | 3,0 |
| | Phenonip | 0,5 |
| | Wasser | 69,3 |
| | | |
| C | Carbomer | 0,2 |
| | Isopropyl Palmitate | 0,8 |
| | | |
| D | Mikropigment aus Beispiel 2 | 5,0 |
| | | |
| E | NaOH (10%ig) | nach Bedarf |

### Beispiel 13: O/W-Emulsion

| | % |
|---|---|
| Potassium Cetyl Phosphate | 2,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Glyceryl Stearate | 3,00 |
| Cetyl Alcohol | 1,00 |
| Phenoxyethanol&Parabens | 1,00 |
| Octyl Methoxycinnamate | 5,00 |
| Dimethicone | 0,10 |
| Deionisiertes Wasser | 64,15 |
| Carbomer (Carbopol 981) | 0,10 |
| Glycerin | 3,00 |
| NaOH (10%ig) | 1,00 |
| Mikropigment aus Beispiel 1 | 4,00 |

### Beispiel 14: O/W-Emulsion:

| | % |
|---|---|
| Cetearyl Alcohol & Dicetyl Phosphate & Ceteth-10 Phosphate | 6,00 |
| Caprylic/Capric Triglyceride | 5,00 |
| Cetearyl Isononanoate | 5,00 |
| C12-15 Alkyl Benzoate | 5,00 |
| Phenoxyethanol & Parabens | 1,00 |
| Octyl Methoxycinnamate | 5,00 |
| Dimethicone | 0,20 |
| | |
| deionisiertes Wasser | 64,70 |
| Carbomer (Carbopol 981) | 0,10 |
| Glycerin | 3,00 |
| NaOH (10%ig) | 0,65 |
| | |
| Mikropigment aus Beispiel 3 | 4,00 |

### Beispiel 15: O/W-Emulsion:

| | % |
|---|---|
| lsopropylmyristate&Trilaureth-4 Phosphate | 5,00 |
| Tricontanyl PVP | 1,00 |
| Caprylic/Capric Triglyceride | 5,00 |
| Cetearyl Isononanoate | 2,00 |
| C12-15 Alkyl Benzoate | 5,00 |
| Glyceryl Stearate | 2,00 |
| Cetyl Alcohol | 1,00 |
| Phenoxyethanol&Parabens | 1,00 |
| Octyl Methoxycinnamate | 5,00 |
| Dimethicone | 0,10 |
| | |
| Deionisiertes Wasser | 66,30 |
| Carbomer (Carbopol 981) | 0,10 |
| Glycerin | 3,00 |
| NaOH (10%ig) | 0,50 |
| | |
| Mikropigment aus Beispiel 4 | 4,00 |

### Beispiel 16: O/W-Emulsion

| | % |
|---|---|
| Sodium Stearyl Lactate Tricontanyl PVP | 1,50 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Glyceryl Stearate | 3,50 |
| Cetyl Alcohol | 2,00 |
| Phenoxyethanol&Parabens | 1,00 |
| Octyl Methoxycinnamate | 5,00 |
| Dimethicone | 0,20 |
| | |
| Deionisiertes Wasser | 63,60 |
| Carbomer (Carbopol 981) | 0,10 |
| Glycerin | 3,00 |
| NaOH (10%ig) | 0,10 |
| | |
| Mikropigment aus Beispiel 6 | 4,00 |

### Beispiel 17: O/W-Emulsion

| | % |
|---|---|
| Cetearyl Alkohol & Sodium Cetearyl Sulfate | 5,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octyl Methoxycinnamate | 5,00 |
| Dimethicone | 0,10 |
| | |
| Deionisiertes Wasser | 65,90 |
| Glycerin | 3,00 |
| NaOH (10%ig) | 0,30 |
| | |
| Mikropigment aus Beispiel 9 | 4,00 |

### Beispiel 18: O/W-Emulsion

| | % |
|---|---|
| Lauryl Glucoside & Polyglyceryl-2 Dihydroxystearate & Glycerin | 3,00 |
| Tricontanyl PVP | 1,00 |
| Caprylic/Capric Triglyceride | 4,00 |
| Cetearyl Isononanoate | 4,00 |
| C12-15 Alkyl Benzoate | 5,00 |
| Glyceryl Stearate | 2,00 |
| Cetyl Alcohol | 3,00 |
| Phenoxyethanol & Parabens | 1,00 |
| Octyl Methoxycinnamate | 5,00 |
| Dimethicone | 0,20 |
| | |
| Deionisiertes Wasser | 64,49 |
| Carbomer (Carbopol 981) | 0,10 |
| Glycerin | 3,00 |
| NaOH (10%ig) | 0,21 |
| | |
| Mikropigment aus Beispiel 8 | 4,00 |

### Beispiel 19: O/W-Emulsion:

| | % |
|---|---|
| Cetaryl Glucoside & Cetearyl Alcohole | 4,50 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octyl triazone | 3,00 |
| 4-Methylbenzylidene camphor | 3,00 |
| Dimethicone | 0,20 |
| | |
| Deionisiertes Wasser | 64,65 |
| Steareth-10 Allyl Ether(Acrylates Copolymer | 5,00 |
| Glycerin | 3,00 |
| NaOH (10%ig) | 1,00 |
| | |
| Mikropigment aus Beispiel 2 | 4,00 |

### Beispiel 20: O/W-Emulsion

| | % |
|---|---|
| Cetearyl Glucoside | 5,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octocrylene | 3,00 |
| Octyl Methoxycinnamate | 4,00 |
| Dimethicone | 0,20 |
| | |
| Deionisiertes Wasser | 63,15 |
| Carbomer (Carbopol 981) | 0,50 |
| Glycerin | 3,00 |
| NaOH (10%ig) | 0,15 |
| | |
| Mikropigment aus Beispiel 2 | 4,00 |

### Beispiel 21: O/W-Emulsion:

| | % |
|---|---|
| Polyglyceryl-10 Petastearate & Behenyl Alcohol & Sodium Ste- | 2,50 |
| aroyl Laurate | |
| Caprylic/Capric Triglyceride | 5,00 |
| Cetearyl Isononanoate | 5,00 |
| C12-15 Alkyl Benzoate | 5,00 |
| Glyceryl Stearate | 3,00 |
| Cetearyl Alcohol | 2,00 |
| Phenoxyethanol&Parabens | 1,00 |
| Octyl Methoxycinnamate | 5,00 |
| Dimethicone | 0,20 |
| | |
| Deionisiertes Wasser | 64,75 |
| Carbomer (Carbopol 981) | 0,15 |
| Glycerin | 3,00 |
| NaOH (10%ig) | 0,40 |
| Mikropigment aus Beispiel 9 | 4,00 |

### Beispiel 22: O/W-Emulsion:

| | % |
|---|---|
| Palmitic Acid & Stearic Acid | 1,80 |
| Glyceryl Stearate SE | 3,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Glyceryl Stearate | 0,50 |
| Phenoxyethanol & Parabens | 1,00 |
| Octyl dimethyl PABA | 5,00 |
| Dimethicone | 0,10 |
| Deionisiertes Wasser | 64,15 |
| Carbomer (Carbopol 981) | 0,10 |
| Glycerin | 3,00 |
| NaOH (10%ig) | 0,50 |
| Mikropigment aus Beispiel 1 | 4,00 |

### Beispiel 23: O/W-Emulsion:

| | % |
|---|---|
| Glyceryl Stearate & PEG 100 Stearate | 3,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Cetearyl Alcohol | 3,00 |
| Phenoxyethanol&Parabens | 1,00 |
| Octyl Methoxycinnamate | 5,00 |
| Dimethicone | 0,10 |
| Deionisiertes Wasser | 64,60 |
| Carbomer (Carbopol 981) | 0,10 |
| Glycerin | 3,00 |
| NaOH (10%ig) | 0,20 |
| Mikropigment aus Beispiel 3 | 4,00 |

### Beispiel 24: O/W-Emulsion:

| | % |
|---|---|
| Steareth-2 | 2,50 |
| Steareth-21 | 1,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Cetyl Alcohol | 1,00 |
| Phenoxyethanol&Parabens | 1,00 |
| Methyl anthranilate | 3,00 |
| Octyl Methoxycinnamate | 4,00 |
| Dimethicone | 0,10 |
| Deionisiertes Wasser | 63,95 |
| Carbomer (Carbopol 981) | 0,20 |
| Glycerin | 3,00 |
| NaOH (10%ig) | 0,25 |
| | |
| Mikropigment aus Beispiel 4 | 4,00 |

### Beispiel 25: O/W-Emulsion:

| | % |
|---|---|
| Glyceryl Stearate&Cetareth-20 & Cetareth-12&Cetaryl Alco- | 5,00 |
| hol&Cetyl Palmitate | |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| 4-Methylbenzylidene camphor | 5,00 |
| Dimethicone | 0,10 |
| deionisiertes Wasser | 65,60 |
| Carbomer (Carbopol 981) | 0,10 |
| Glycerin | 3,00 |
| NaOH (10%ig) | 0,20 |
| Mikropigment aus Beispiel 3 | 4,00 |

### Beispiel 26: O/W-Emulsion

| | % |
|---|---|
| Octyldecyl Phosphate | 3,00 |
| Tricontanyl PVP | 1,00 |
| Caprylic/Capric Triglyceride | 5,00 |
| Cetearyl Isononanoate | 5,00 |
| C12-15 Alkyl Benzoate | 5,00 |
| Phenoxyethanol&Parabens | 1,00 |
| Octyl methoxycinnamate | 5,00 |
| Dimethicone | 0,10 |
| | |
| Deionisiertes Wasser | 64,50 |
| Sodium Cocoyl Glutamate | 0,60 |
| Steareth-10 Allyl Ether/ Acrylates Copolymer | 0,50 |
| Glycerin | 3,00 |
| NaOH (10%ig) | 2,30 |
| Mikropigment aus Beispiel 4 | 4,00 |

### Beispiel 27: O/W-Emulsion:

| | % |
|---|---|
| Polyglyceryl-3 Methyl Glucose Distearate | 2,00 |
| Tricontanyl PVP | 1,00 |
| Tocopherol&Ascorbyl Palmitate&Ascorbic Acid&Citric Acid&PEG-8 | 0,05 |
| Decyl Oleate | 4,50 |
| Isopropyl Palmitate | 6,00 |
| Caprylic/Capric Triglyceride | 5,00 |
| Glyceryl Stearate | 1,00 |
| Cetearyl Alcohol | 1,00 |
| 2-[(2,4-methoxy)-phenyl]-4,6-bis-[(2-hydroxy-4-methoxy)-phenyl]-(1,3,5)-triazine | 2,00 |
| Octyl Methoxycinnamate | 3,00 |
| deionisiertes Wasser | 63,12 |
| Phenoxyethanol&Parabens | 0,80 |
| Propylene Glycol | 3,00 |
| Carbomer (Carbopol 981) | 0,20 |
| NaOH (10%ig) | 0,33 |
| Scleroglucan | 1,00 |
| Mikropigment aus Beispiel 2 | 3,00 |
| Titanium Dioxide | 3,00 |

### Beispiel 28: O/W-Emulsion

| | % |
|---|---|
| Methyl Glucose Sequistearate | 2,50 |
| Tricontanyl PVP | 1,00 |
| Tocopherol&Ascorbyl Palmitate&Ascorbic Acid&Citric Acid&PEG-8 | 0,05 |
| Decyl Oleate | 4,00 |
| Isopropyl Palmitate | 6,00 |
| Caprylic/Capric Triglyceride | 5,00 |
| Glyceryl Stearate | 1,00 |
| Cetearyl Alcohol | 1,00 |
| 2-[(2,4-methoxy)-phenyl]-4,6-bis-[(2-hydroxy-4-methoxy)-phenyl]-(1,3,5)-triazine | 2,00 |
| Octyl Methoxycinnamate | 5,00 |
| | |
| deionisiertes Wasser | 63,12 |
| Phenoxyethanol&Parabens | 0,80 |
| Carbomer (Carbopol 981) | 0,20 |
| Glycerin | 3,00 |
| NaOH (10%ig) | 0,33 |
| Scleroglucan | 1,00 |
| Mikropigment aus Beispiel 1 | 4,00 |

### Beispiel 29: Lippenpflegemittel

| | % |
|---|---|
| Glycerin | 10,00 |
| PEG-45&Dodecyl Glycerol Copolymer | 1,50 |
| Quaternium-18 Bentonit | 2,00 |
| Microkristallines Wachs | 2,00 |
| Bienenwachs | 2,00 |
| Glyceryl Stearate SE | 53,00 |
| Pentaerythrithil Stearat&Caprate&Caprylat Adipate | 4,00 |
| Castor Oil | 4,00 |
| Methylene Bis-benzotriazolyl Tetramethylbutylphenol | 5,00 |
| Mikropigment aus Beispiel 2 | 5,00 |
| Titanium Dioxide | 5,00 |
| Zink Oxide | 5,00 |
| Octyl Methoxycinnamate | 4,00 |
| Eucerinum anhydricum | ad 100 |

### Beispiel 30: W/O-Emulsion

| | % |
|---|---|
| PEG-30 Dipolyhydroxystearate | 2,00 |
| Isostearyl Alcohol | 20,00 |
| Isostearic Acid | 10,00 |
| Octyl Triazone | 3,00 |
| | |
| deionisiertes Wasser | 58,75 |
| Glycerin | 5,00 |
| Methylparaben | 0,17 |
| Propylparaben | 0,03 |
| MgSO₄x7H₂O | 0,75 |
| | |
| Mikropigment aus Beispiel 2 | 4,00 |

### Beispiel 31: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | Octyl Methoxycinnamate | 5,0 |
| | | |
| B | Glycerol | 3,0 |
| | Phenonip | 0,5 |
| | deion. Wasser | 62,9 |
| | | |
| C | Carbomer 141 | 0,2 |
| | Isopropyl Palmitate | 0,8 |
| | | |
| D | 50%ige Suspension aus Beispiel 8 | 8,0 |
| | | |
| E | NaOH (10%) | nach Bedarf |

### Beispiel 32: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | | |
| B | Glycerol | 3,0 |
| | Phenonip | 0,5 |
| | deionis. Wasser | 62,9 |
| | | |
| C | Carbomer 141 | 0,2 |
| | Isopropyl Palmitate | 0,8 |
| | | |
| D | Suspension aus Beispiel 2 | 6,0 |
| | | |
| E | NaOH (10%) | nach Bedarf |

### Beispiel 33: (O/W-Emulsion)

| | | % |
|---|---|---|
| A | Polyglyceryl-3 Methylglucose Distearate | 2,0 |
| | Decyl Oleate | 5,7 |
| | Isopropyl Palmitate | 5,0 |
| | Caprylic/Capric Triglyceride | 6,5 |
| | Octyl Triazone | 2,0 |
| | | |
| B | Glycerol | 3,0 |
| | Phenonip | 0,5 |
| | Wasser | 62,3 |
| | | |
| C | Carbomer 141 | 0,2 |
| | Isopropyl Palmitate | 0,8 |
| | | |
| D | 2,2'-Methylene-bis-(6-(2H-benzotriazol-2-yl)-4-(1,1,3,3- | 8,0 |
| | tetramethylbutyl)phenol-Mikropigment-Suspension | |
| | (50%) | |
| | Octyl Triazone-Mikropigment-Suspension (50%ig) | 4,0 |
| | | |
| E | NaOH (10%) | nach Bedarf |

### Beispiel 34: O/W-Emulsion

| | | % |
|---|---|---|
| A | Polyglyceryl-3 Methylglucose Distearate | 2,0 |
| | Decyl Oleate | 5,7 |
| | Isopropyl Palmitate | 5,0 |
| | Octyl Triazone | 2,0 |
| | Caprylic/Capric Triglyceride | 6,5 |
| | | |
| B | Glycerol | 3,0 |
| | Phenonip | 0,5 |
| | Wasser | 68.3 |
| | | |
| C | Carbomer 141 | 0,2 |
| | Isopropyl Palmitate | 0,8 |
| | | |
| D | Mikropigment aus Beispiel 2 | 6,0 |
| | | |
| E | NaOH (10%) | nach Bedarf |

### Beispiel 35: W/O-Emulsion

| | % |
|---|---|
| PEG-30 Dipolyhydroxystearate (Arlacel P 135®) | 3,00 |
| PEG-22/ Dodecyl Glycol Copolymer (Elfacos ST 37®) | 1,00 |
| Microkrystallines Wachs | 1,00 |
| Hydrogenated Castor Öl | 0,50 |
| Magnesium Stearate | 1,00 |
| Octyl Stearate | 15,00 |
| Coco Glycerides | 2,00 |
| Mineral Öl | 3,00 |
| Phenoxyethanol&Parabens | 1,00 |
| Octyl Methoxycinnamate | 5,00 |
| Dimethicone | 0,10 |
| Wasser | 54,40 |
| Magnesium Sulphate (MgSO₄ x 7 H₂O) | 1,00 |
| Propylene Glycol | 4,00 |
| 50%ige Suspension aus Beispiel 3 | 8,00 |

### Beispiel 36: W/O -Emulsion

| | % |
|---|---|
| Methoxy PEG-22/Dodecyl Glycol Copolymer (Elfacos E 200®) | 3,00 |
| PEG-22/ Dodecyl Glycol Copolymer (Elfacos ST 37®) | 3,00 |
| Hydroxyoctacosanyl Hydroxystearate (Elfacos C 26®) | 3,00 |
| Octyl Stearate | 15,00 |
| Coco Glycerides | 2,00 |
| Mineral Oil | 3,00 |
| Phenoxyethanol&Parabens | 1,00 |
| 4-Methylbenzylidene Camphor | 3,00 |
| Dioctyl Butamido Triazone | 3,00 |
| Dimethicone | 0,20 |
| Wasser | 53,00 |
| Phenylbenzimidazol Sulphonsäure | 3,00 |
| Magnesium Sulphate (MgSO₄ x 7 H₂O) | 0,80 |
| Propylene Glycol | 4,00 |
| Mikropigment aus Beispiel 5 | 3,00 |

### Beispiel 37: W/O -Emulsion

| | % |
|---|---|
| Polyglyceryl-2 Dipolyhydroxystearate (Dehymuls PGPH®) | 2,00 |
| PEG-30 Dipolyhydroxystearate (Arlacel P 135®) | 2,00 |
| Hydroxyoctacosanyl Hydroxystearate (Elfacos C 26®) | 2,00 |
| Zink Stearate | 1,00 |
| Octyl Stearate | 15,00 |
| Coco Glycerides | 2,00 |
| Mineral Oil | 3,00 |
| Phenoxyethanol & Parabens | 1,00 |
| 2,4-Bis-{[4-(2-Ethyl-hexyloxy)-2-hydroxy]-phenyl}-6-(4-methoxyphenyl)1,3,5)-triazine | 2,00 |
| Octyl Salicylate | 3,00 |
| Dimethicone | 0,20 |
| Wasser | 56,70 |
| Magnesium Sulphate (MgSO₄ x 7 H₂O) | 1,00 |
| Propylene Glycol | 4,00 |
| Mikropigment aus Beispiel 6 | 5,00 |

### Beispiel 38: W/O -Emulsion

| | % |
|---|---|
| Polyglyceryl-2-Dipolyhydroxystearate (Dehymuls PGPH®) | 3,00 |
| Glyceryl Oleate (Monomuls 90-O 18®) | 1,00 |
| Caprylic / Capric Triglyceride | 6,00 |
| Octyldodecanol | 6,00 |
| Cetearyl Isononaoate | 5,00 |
| Tocopheryl Acetate | 1,00 |
| Cera alba | 1,20 |
| Glycerin (86 %) | 5,00 |
| Phenonip | 0,50 |
| Octyl Methoxycinnamate | 4,00 |
| Octyl Triazone | 3,00 |
| Mikropigment aus Beispiel 3 | 5,00 |
| Wasser | ad 100 |

### Beispiel 39: W/O -Emulsion

| | % |
|---|---|
| Polyglyceryl-2-Dipolyhydroxystearate (Dehymuls PGPH®) | 3,00 |
| Glyceryl Oleate (Monomuls 90-O 18®) | 1,00 |
| Caprylic / Capric Triglyceride | 6,00 |
| Octyldodecanol | 6,00 |
| Cetearyl Isononaoate | 5,00 |
| Octyl Methoxycinnamate | 3,00 |
| Tocopheryl Acetate | 1,00 |
| Cera alba | 1,20 |
| Glycerin (86 %) | 5,00 |
| Phenonip | 0,50 |
| Mikropigment aus Beispiel 10 | 5,00 |
| Wasser | ad 100 |

### Beispiel 40: O/W-Emulsion

| | % |
|---|---|
| Tego Care CG 90 (Goldschmidt AG) | 6,00 |
| Cetearyl Alcohol | 1,50 |
| Glycerylstearat | 0,50 |
| Octyldecanol | 7,00 |
| Capric/Caprylic Triglyceride | 5,00 |
| Cetearylisononaoat | 6,00 |
| Octyl Methoxycinnamate | 3,00 |
| Deionisiertes Wasser | 51,14 |
| Carbomer | 0,20 |
| NaOH (45%ig) | 1,13 |
| Glycerin | 5,00 |
| Methylparaben | 0,17 |
| Propylparaben | 0,03 |
| Terephthalyden-dibornan-sulphonsäure | 1,50 |
| Mikropigment aus Beispiel 5 (50%ige Suspension) | 12,00 |

### Beispiel 41: O/W-Mikroemulsion

| | % |
|---|---|
| Ceteareth-12 | 8,0 |
| Cetearyl Alcohol | 4,0 |
| Cetearylisononanoat | 20,0 |
| Butyl Methoxydibenzoylmethan | 2,0 |
| Deionisiertes Wasser | ad 100,0 |
| Carbomer | 0,2 |
| Konservierungsmittel | nach Bedarf |
| Magnesium Sulphate (MgSO₄ x 7 H₂O) | 3,0 |
| Mikropigment aus Beispiel 9 (50%ige Suspension) | 8,0 |

### Beispiel 42: O/W/O-Emulsion

| | % |
|---|---|
| Polyglyceryl-2-polyhydroxystearat | 5,0 |
| Mineralöl | 12,5 |
| Stearinsäure | 2,0 |
| Cetearylisononaoat | 12,5 |
| Methylbenzylidene Camphor | 2,0 |
| Homosalate | 2,0 |
| Deionisiertes Wasser | ad 100,0 |
| Carbomer | 0,2 |
| Konservierungsmittel | nach Bedarf |
| NaOH | nach Bedarf |
| Mikropigment aus Beispiel 2 (50%ige Suspension) | 8,0 |

### Beispiel 43: O/W -Emulsion

| | % |
|---|---|
| Glycerinstearat/Polyethylenglycol(MG100)-stearat | 3,0 |
| Cetyl-/Stearylalkohol-20EO (Eumulgin B 2) | 1,0 |
| Cetyl-/Stearylalkohol (Lanette O) | 2,0 |
| Capryl-/Caprin-triglycerid (Myritol 318) | 4,0 |
| Dicaprylylether | 6,0 |
| Mineralöl und Quarternium-18 Hectorite | 3,0 |
| Glycerinstearat, Cetyl-/Stearylalkohol, Cetylpalmitat, Kokosglyceride (Cutina CBS) | 2,0 |
| 4-Methylbenzylidene Camphor | 1,0 |
| Octyl Triazone | 2,0 |
| Deionisiertes Wasser | ad 100,0 |
| Glycerin, 85%ig | 3,0 |
| Konservierungsmittel | nach Bedarf |
| Magnesiumaluminiumsilikat (Vegum Ultra) | 0,3 |
| NaOH | nach Bedarf |
| Mikropigment aus Beispiel 2 (50%ige Suspension) | 10,0 |

### Beispiel 44:

In das Sun Care-Produkt "Sensitive Skin" (children) von Lancaster (Monaco), charakterisiert durch folgende Inhaltsstoffe: TiO₂, ZnO sowie Aqua, Didecene, Glycerine, Cyclomethicone, Shea Butter, Sweet Almond Oil, Polyglycerin-4, Urea, Aluminum Starch, Octenylsuccinate, Alumina, Parfum, MgSO₄, Silica, NaCl, Tocopheryl acetate, Caffeine, PVP/Eicosene Copolymer, Shellac, Simethicone, Phenoxyethanol, Na-Lactate, Methylsilanol, Menthyl Lactate, Allantoin, Bisabolol, Glycine, Panthenol, Propylene Glycol, Stoneroot Extract, Lecithin, Algae Extract, Methyldibromo Glutaronitrile, PVP, Citric Acid, Copper Gluconate, Ascorbic Acid, Ascorbyl Palmitate, PEG-8, Tocopherol, Acerola, Aloe Barbadensis Gel, Melanin, Alcohol denat. Dimethicone, Guar Hydroxypropyltrimonium Chloride, Dextrin, Glycoproteins Iron oxides, wurden 4% mikronisiertes 2,2'-Methylen-bis-[6-(2H-benztriazol-2-yl)-4-(1,1,3,3-tetramethylbutyl)-phenol (d50 = 200 nm) nachträglich zugemischt. Der ursprüngliche SPF von 15 erhöhte sich dadurch auf 25 und steigerte sich nach mehrtägiger Lagerung nochmals auf den SPF von 31.

### Beispiel 45:

In die Sun Milk "Aktive Sun Care Sensitive Skin" von Marbert Cosmetics, Düsseldorf,, charakterisiert durch folgende Inhaltsstoffe: TiO₂, Benzophenone 3, Isoamyl p-Methoxycinnamate, sowie Aqua, C₁₂₋₁₅ Alkyl benzoate, Caprylic/Capric Triglyceride, Cyclomethicone, Glycerine, Glyceryl Stearate, Cetearyl Alkohol, Tocopheryl acetate, Stearic Acid, Palmitic Acid, Parfum, Na-Cocoyl Lactylate, Xanthan Gum, Bisabolol, DMDM Hydantoin, PVM/MA Decadiene Crosspolymer, Polyhydroxystearic acid, Alumina, NaOH, Glucose, lodopropynyl Butylcarbamate, Carrageenan, Silica und Glucuronic acid, wurden 4% mikronisiertes 2,2'-Methylen-bis-[6-(2H-benztriazol-2-yl)-4-(1,1,3,3-tetramethylbutyl)-phenol (d50 = 200 nm) nachträglich zugemischt. Der ursprüngliche SPF von 6 erhöhte sich dadurch auf 13 und steigerte sich nach mehrtägiger Lagerung nochmals auf den SPF von 16.

### Beispiel 46:

In die Sonnenschutzemulsion "Delial Sonnenmilch 10" von Sara Lee, Düsseldorf, , charakterisiert durch folgende Inhaltsstoffe: Octyl Methoxycinnamate, Na-Phenylbenzimidazole Sulfonate, Butyl Methoxy Dibenzoylmethane sowie Aqua, Paraffinum liquidum, Alkohol denat., Isopropyl Palmitate, Glycerine, Cetearyl Alkohol, Glyceryl Stearat SE, Tocopheryl acetate, Phytantriol, Ascorbyl Palmitate, PEG-40 Castor Oil, Na-Cetearyl Sulfate, Dimethicone, Na-Carbomer, Na₂-EDTA und Parfum, wurden 4% mikronisiertes 2,2'-Methylen-bis-[6-(2H-benztriazol-2-yl)-4-(1,1,3,3-tetramethylbutyl)-phenol (d50 = 200 nm) nachträglich zugemischt. Der ursprüngliche SPF von 10 erhöhte sich dadurch auf 18 und steigerte sich nach mehrtägiger Lagerung nochmals auf den SPF von 28.

### Beispiel 47:

In die Sonnenschutzformulierung "Ambre Solaire" SPF 12 von Laboratoires Garnier, Paris/Karlsruhe, charakterisiert durch folgende Inhaltsstoffe: TiO₂, Octocrylene, Butyl Methoxy Dibenzoylmethane, Terephthalylidene dicamphor sulfonic acid sowie Aqua, Cyclopentasiloxane, Glycerine, Propylene glycol, Isohexadecane, Stearic acid, Octyl palmitate, Stearyl heptanoate, PVP/Eicosene Copolymer, K-Cetyl Phosphate, Buxus chinensis, Tocopheryl acetate, Hydroxypropyl Methylcellulose, Phenoxyethanol, Stearylcaprylate, PEG-100 Stearate, Ethylparaben, Triethanolamin, Dimethiconol, Dimethicone, Propylparaben, Acrylates/C₁₀₋₃₀-Alkyl acrylate crosspolymer, Na₂-EDTA, Butyrospermum parkii, Cetylalkohol, Methylparaben, Butylparaben, BHT, Aluminium hydroxide, Glycerylstearat, wurden 4% mikronisiertes 2,2'-Methylen-bis-[6-(2H-benztriazol-2-yl)-4-(1,1,3,3-tetramethylbutyl)-phenol (d50 = 200 nm) nachträglich zugemischt. Der ursprüngliche SPF von 12 erhöhte sich dadurch auf 18 und steigerte sich nach mehrtägiger Lagerung nochmals auf den SPF von 28.

### Beispiel 48:

In die Sonnenschutzformulierung "Ambre Solaire" SPF 6 von Laboratoires Garnier, Paris/Karlsruhe, charakterisiert durch folgende Inhaltsstoffe: TiO₂, Octocrylene, Butyl Methoxy Dibenzoylmethane, Terephthalidene dicamphor sulphonic acid sowie Aqua, Cyclomethicone, Glycerine, Propylene glycol, Isohexadecane, Stearic acid, Octyl palmitate, Stearyl heptanoate, PVP/Eicosene Copolymer, K-Cetyl Phosphate, Buxus chinensis, Tocopheryl acetate, Hydroxypropyl Methylcellulose, Phenoxyethanol, Stearyl caprylate, PEG-100 Stearate, Ethylparaben, Triethanolamin, Dimethiconol, Dimethicone, Propylparaben, Acrylates/C₁₀₋₃₀-Alkyl acrylate crosspolymer, Na₂-EDTA, Butyrospermum parkii, Cetylalkohol, Methylparaben, Butylparaben, BHT, Aluminium hydroxide, Glycerylstearat und Parfum, wurden 4% mikronisiertes 2,2'-Methylen-bis-[6-(2H-benztriazol-2-yl)-4-(1,1,3,3-tetramethylbutyl)-phenol (d50 = 200 nm) nachträglich zugemischt. Der ursprüngliche SPF von 6 erhöhte sich dadurch auf 16 und steigerte sich nach mehrtägiger Lagerung nochmals auf den SPF von 21.

### Beispiel 49: Verhinderung der Zunahme der Hautbräunung durch einen mikronisierten UV-Absorber Methylene Bis-benzotriazolyl Tetramethylbutylphenol

### Methode:

20 Freiwillige asiatischer Herkunft ersten Grades (Vater und Mutter) ohne direkte Sonnenexposition während der vergangenen 3 Monate werden nach Aufklärung über die Studie, Einverständniserklärung und Erfüllung der Aufnahmebedingungen zwei mal täglich über drei Wochen mit einer Creme, enthaltend 2,2'-Methylene-bis-(6(2H-benztriazol-2-yl)-4-(1,1,3,3-tetramethylbuthyl)phenol bzw. mit einer Placebocreme auf den Prüfstellen am Oberschenkel behandelt .
Die Freiwilligen werden an den Prüfstellen 3x wöchentlich mit 0.2 bis 0.5 MED UVAB am Oberschenkel bestrahlt.
Die erste Applikation der Präparate erfolgt nach der ersten Bestrahlung. Ausgewertet und bestrahlt wird jeweils nach dem Auftragen der Prüfprodukte. Vergleichbare unbehandelte bestrahlte, bzw. unbehandelte nicht bestrahlte Flächen dienen als Referenz.
Die Farbwerte der Prüffelder werden jeweils mittels einer Minolta CM-508i Kamera als La*b*-Werte gemäss DIN 5033, ISO 7724/1, JIS Z8722,dokumentiert.
Die Farb- und Helligkeitsänderungen werden bei jedem Probanden bestimmt und als Differenz zwischen der jeweiligen Hautfarbe der unbehandelten, unbestrahlten Referenzfläche und den Prüfflächen ermittelt. Diese Werte werden über alle Probanden gemittelt und als L*a*b*- Werte angegeben.

### Prüfpräparate:

(A): Zusammensetzung, enthaltend 6% 2,2'-Methylene-bis-(6(2H-benztriazol-2-yl)-4-(1,1,3,3-tetramethylbuthyl)phenol), Wasser, Octyl stearate, Coco glycerides, Propylene glycol, Methoxy-PEG-22/Dodecyl glycol copolymer, PEG-22/Dodecyl glycol copolymer, Hydroxyoctacosanyl hydroxystearate, Mineral oil, Phenoxyethanol&Parabens, Magnesium sulfate hepta-hydrate, Dimethicone, Allantoin.
(B): Zusammensetzung, enthaltend 3% 2,2'-Methylene-bis-(6(2H-benztriazol-2-yl)-4-(1,1,3,3-tetramethylbutyl)phenol, Wasser, Octyl stearate, Coco glycerides, Propylene glycol, Methoxy-PEG-22/Dodecyl glycol copolymer, PEG-22/Dodecyl glycol copolymer, Hydroxyoctacosanyl hydroxystearate, Mineral oil, Phenoxyethanol&Parabens, Magnesium sulfate hepta-hydrate, Dimethicone, Allantoin.
(C): Placebo enthaltend Wasser, Octyl stearate, Coco glycerides, Propylene glycol, Methoxy-PEG-22/Dodecyl glycol copolymer, PEG-22/Dodecyl glycol copolymer, Hydroxyoctacosanyl hydroxystearate, Mineral oil, Phenoxyethanol&Parabens, Magnesium sulfate heptahydrate, Dimethicone, Allantoin.

L*a*b*-Werte im Vergleich zu unbestrahlter Haut nach mehrfacher UVAB Bestrahlung (3 x wöchentlich) und bei Applikation der Zusammensetzungen (A) und (B).

| Präparat | Helligkeit L* | | | Rotanteil a* | | | Gelbanteil b* | | |
|---|---|---|---|---|---|---|---|---|---|
| Anzahl Bestrahlungen | 3 | 6 | 9 | 3 | 6 | 9 | 3 | 6 | 9 |
| Placebo | -5.32 | -12.01 | -14.01 | 4.90 | 2.33 | 0.52 | 3.09 | 6.82 | 7.93 |
| (B) | -1.05 | -5.23 | -7.13 | 0.45 | 0.77 | -0.03 | 1.11 | 2.63 | 3.49 |
| (A) | 2.18 | 8.26 | 11.40 | 0.24 | 0.45 | 0.39 | -0.38 | -0.69 | -0.21 |
| bestrahlt unbehandelt | -5.19 | -12.38 | -14.55 | 5.13 | 1.77 | -0.19 | 2.64 | 6.39 | 7.44 |

### Kommentar zu den Ergebnissen:

### Helligkeit

Während die Placebo behandelte und die unbehandelten bestrahlten Flächen etwa gleichermassen an Helligkeit abnehmen, das heisst dunkler werden, wirkt sich dieser Effekt bei Applikation der Zusammensetzung (B) über die Zeit wesentlich geringer aus. Bei Applikation der Zusammensetzung (A) wird ein Hellerwerden der Haut festgestellt.

### Rötung

Der Rotanteil der bestrahlten Haut ist nach 3-maliger Bestrahlung am intensivsten und fällt bei Ende der Bestrahlungen auf den Normalwert zurück. Die Steigerung des Rotanteils entspricht dem Verlauf eines UV-induzierten Erythems das bei Applikation der Zusammensetzungen (A) oder (B) nur in geringem Masse auftritt.

### Gelbanteil

Der Gelbanteil nimmt sowohl bei Applikation von Placebo als auch in der unbehandelten bestrahlten Kontrollfläche zu. Die Zunahme ist bei Applikation der Zusammensetzung (B) weit geringer und wird bei Applikation der Zusammensetzung (A) verhindert.

## Patentansprüche

1. Verwendung von mikronisierten organischen UV-Filtern zur Bräunungsverhinderung und Aufhellung der menschlichen Haut und Haare.

2. Verwendung nach Anspruch 1, dadurch gekennzeichnet, dass die organischen UV-Filter ausgewählt sind aus Triazin- oder Benzotriazol-Derivaten, eine Vinylgruppe enthaltenden Amiden, Zimtsäurederivaten, sulfonierten Benzimidazolen, Fischerbase-Derivaten, Diphenylmalonsäuredinitrilen, Oxalylamiden, Campherderivaten, Diphenylacrylaten, Paraaminobenzoesäure (PABA) und deren Derivaten, Salicylaten und Benzophenonen.

3. Verwendung nach Anspruch 1 oder 2, dadurch gekennzeichnet, dass die organischen UV-Filter ausgewählt sind aus Triazinderivaten der Formel 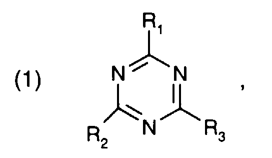 worin
R₁, R₂ und R₃, unabhängig voneinander Wasserstoff; OH; C₁-C₁₈-Alkoxy; -NH₂; -NH-R₄; -N(R₄)₂; -OR₄,
R₄ C₁-C₅-Alkyl; Phenyl; Phenoxy; Anilino; Pyrrolo, worin Phenyl, Phenoxy, Anilino oder Pyrrolo nicht substituiert oder durch einen, zwei oder drei OH-Gruppen, Carboxy, -CO-NH₂, C₁-C₅-Alkyl oder C₁-C₅-Alkoxy substituiert sein können; eine Methylidencampher-Gruppe; eine Gruppe der Formel -(CH=CH)ₘC(=O)-OR₄; eine Gruppe der Formel 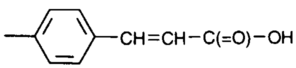 oder die entsprechenden Alkalimetall-, Ammonium-, Mono-, Di- oder Tri-C₁-C₄-Alkylammonium-, Mono-, Di- oder Tri-C₂-C₄-Alkanolammonium- Salze, oder deren C₁-C₃-Alkylester; oder einen Rest der Formel 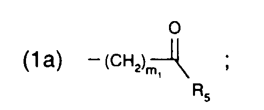
R₅ Wasserstoff; nicht substituiertes oder durch eine oder mehrere OH-Gruppen substituiertes C₁-C₅-Alkyl; C₁-C₅-Alkoxy; Amino; Mono- oder Di-C₁-C₅-Alkylamino; M; einen Rest der Formel 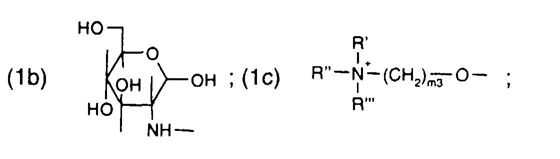 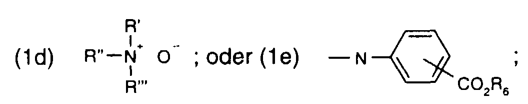 worin
R', R" und R"' unabhängig voneinander nicht substituiertes oder durch eine oder mehrere OH-Gruppen substituiertes C₁-C₁₄-Akyl;
R₆ Wasserstoff; M; C₁-C₅-Alkyl; oder einen Rest der Formel -(CH₂)_{m₂}-O-T₁ ;
M ein Metallkation;
T₁ Wasserstoff; oder C₁-C₈-Alkyl;
m 0 oder 1
m₂ 1 bis 4; und
m₃ 2 bis14;
bedeuten.

4. Verwendung nach Anspruch 1 oder 2, dadurch gekennzeichnet, dass die organischen UV-Filter ausgewählt sind aus Triazinderivaten der Formel 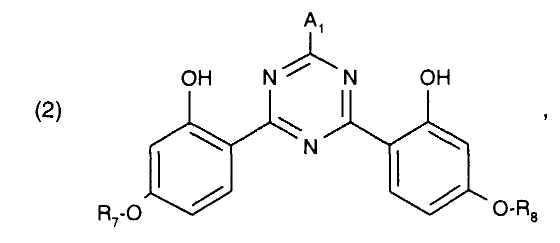 worin
R₇ und R₈, unabhängig voneinander, C₁-C₁₈-Alkyl; C₂-C₁₈-Alkenyl; einen Rest der Formel -CH₂-CH(-OH)-CH₂-O-T₁ ; oder
R₇ und R₈ einen Rest der Formel 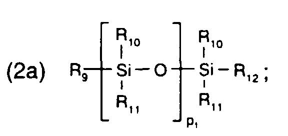
R₉ die direkte Bindung; einen geradkettigen oder verzweigten C₁-C₄-Alkylenrest oder einen Rest der Formel 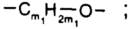
R₁₀, R₁₁ und R₁₂, unabhängig voneinander C₁-C₁₈-Alkyl; C₁-C₁₈-Alkoxy oder einen Rest der Formel 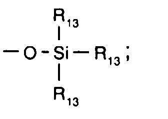
R₁₃ C₁-C₅-Alkyl;
m₁ 1 bis 4;
p₁ 0 bis 5;
A₁ einen Rest der Formel 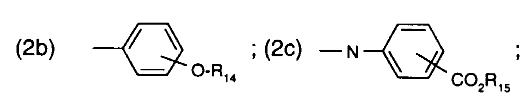 oder der Formel 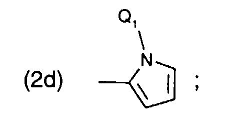
R₁₄ Wasserstoff; C₁-C₁₀-Alkyl, -(CH₂CHR₁₆-O)_{n₁}-R₁₅ ; oder einen Rest der Formel -CH₂-CH(-OH)-CH₂-O-T₁;
R₁₅ Wasserstoff; M; C₁-C₅-Alkyl; oder einen Rest der Formel -(CH₂)_{m₂}-O-(CH₂)_{m₃} -T₁ ;
R₁₆ Wasserstoff; oder Methyl;
T₁ Wasserstoff; oder C₁-C₈alkyl;
Q₁ C₁-C₁₈-Alkyl;
M ein Metallkation;
m₂ und m₃ unabhängig voneinander 1 bis 4; und
n₁ 1 bis 16;
bedeuten.

5. Verwendung nach Anspruch 1 oder 2, dadurch gekennzeichnet, dass die organischen UV-Filter ausgewählt sind aus Triazinderivaten der Formel 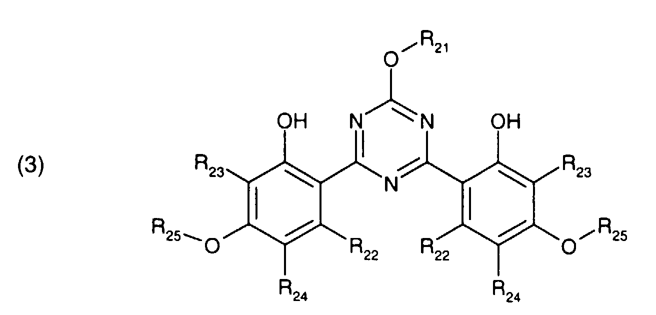 worin
R₂₁ C₁-C₃₀-Alkyl; C₂-C₃₀-Alkenyl; nicht substituiertes oder durch C₁-C₅Alkyl mono- oder polysubstituiertes C₅-C₁₂-Cycloalkyl, C₁-C₅-Alkoxy-C₁-C₁₂-Alkyl; Amino-C₁-C₁₂-Alkyl; C₁-C₅-Monoalkylamino-C₁-C₁₂-Alkyl; C₁-C₅-Dialkylamino-C₁-C₁₂-Alkyl; einen Rest der Formel 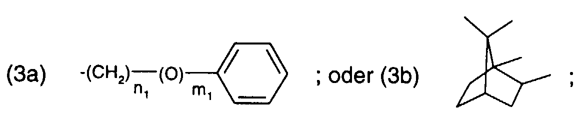 worin
R₂₂, R₂₃ und R₂₄, unabhängig voneinander, Wasserstoff, -OH; C₁-C₃₀-Alkyl, C₂-C₃₀-Alkenyl,
R₂₅ Wasserstoff; oder C₁-C₅-Alkyl;
m₁ 0 oder 1; und
n₁ 1 bis 5;
bedeuten.

6. Verwendung nach Anspruch 1 oder 2, dadurch gekennzeichnet, dass die organischen UV-Filter ausgewählt sind aus Triazinderivaten der Formel 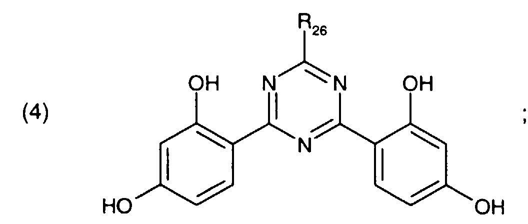 worin
R₂₆ 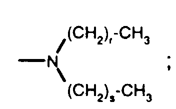 und
r und s unabhängig voneinander 0 bis 20;
bedeuten.

7. Verwendung nach Anspruch 1 oder 2, dadurch gekennzeichnet, dass die organischen UV-Filter ausgewählt sind aus Triazinderivaten der Formel

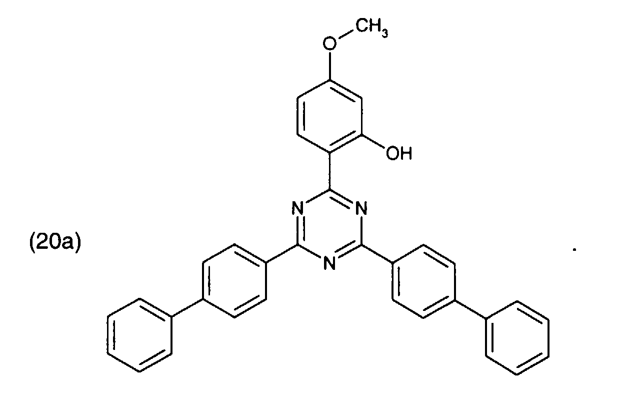

8. Verwendung nach Anspruch 1 oder 2, dadurch gekennzeichnet, dass die organischen UV-Filter ausgewählt sind aus Triazinderivaten der Formel

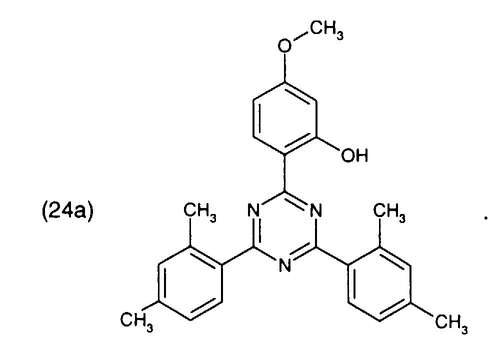

9. Verwendung nach Anspruch 1 oder 2, dadurch gekennzeichnet, dass die organischen UV-Filter ausgewählt sind aus Triazinderivaten der Formel

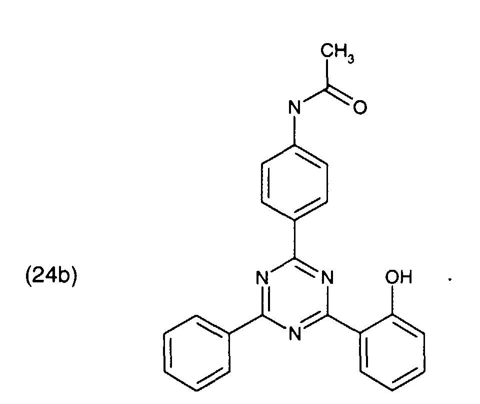

10. Verwendung nach Anspruch 1 oder 2, dadurch gekennzeichnet, dass die organischen UV-Filter ausgewählt sind aus Triazinderivaten der Formel 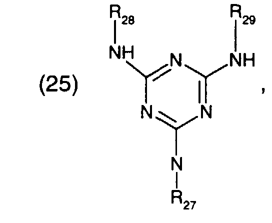 worin
R₂₇, R₂₈ und R₂₉ unabhängig voneinander einen Rest der Formel 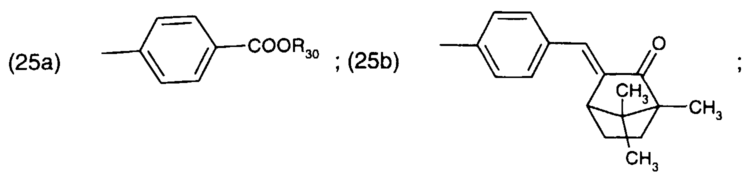 oder 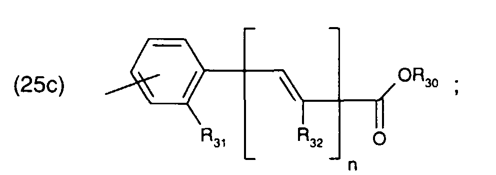
R₃₀ Wasserstoff; Alkali-Metall; eine Ammoniumgruppe -N(R₃₃)₄,
R₃₃ Wasserstoff C₁-C₅-Alkyl; oder einen Polyoxyethylenrest, der 1 bis 10 Ethylenoxideinheiten aufweist und die endständige OH-Gruppe mit einem C₁-C₅-Alkohol verethert sein kann;
R₃₁ Wasserstoff; -OH; oder C₁-C₆-Alkoxy;
R₃₂ Wasserstoff oder -COOR₃₀; und
n 0 oder 1;
bedeuten.

11. Verwendung nach Anspruch 1 oder 2, dadurch gekennzeichnet, dass die organischen UV-Filter ausgewählt sind aus Benzotriazolderivaten der Formel 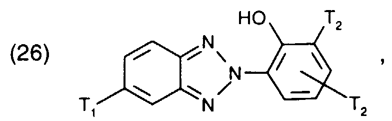 worin
T₁ C₁-C₅-Alkyl oder Wasserstoff; und
T₂ C₁-C₅-Alkyl oder phenylsubstituiertes C₁-C₅-Alkyl;
bedeuten.

12. Verwendung nach Anspruch 1 oder 2, dadurch gekennzeichnet, dass die organischen UV-Filter ausgewählt sind aus Benzotriazolderivaten der Formel 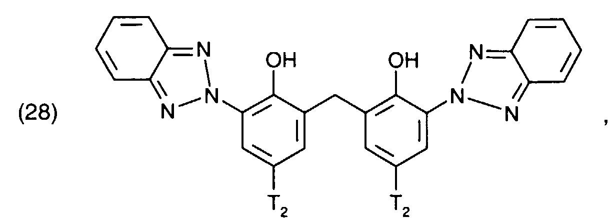 worin
T₂ C₁-C₄-Alkyl oder phenylsubstituiertes C₁-C₅-Alkyl;
bedeutet.

13. Verwendung nach Anspruch 1 oder 2, dadurch gekennzeichnet, dass die organischen UV-Filter ausgewählt sind aus Fischer-Base-Aldehyden der Formel 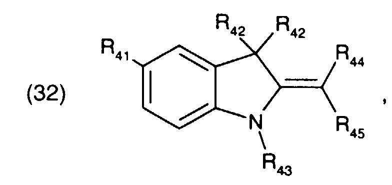 worin
R₄₁ Wasserstoff; C₁-C₅-Alkyl; C₁-C₁₈-Alkoxy; oder Halogen;
R₄₂ C₁-C₈-Alkyl; C₅-C₇-Cycloalkyl; oder C₆-C₁₀-Aryl;
R₄₃ C₁-C₁₈-Alkyl oder ein Rest der Formel 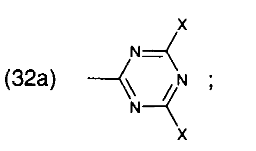
R₄₄ Wasserstoff; oder ein Rest der Formel 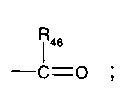
R₄₅ 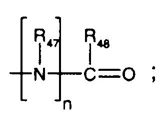 C₁-C₁₈-Alkoxy; oder einen Rest der Formel 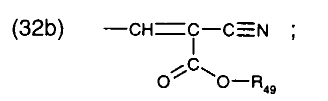
R₄₆ und R₄₇ unabhängig voneinander Wasserstoff; oder C₁-C₅-Alkyl;
R₄₈ Wasserstoff; C₁-C₅-Alkyl; C₅-C₇-Cycloalkyl; Phenyl; Phenyl-C₁-C₃-Alkyl;
R₄₉ C₁-C₁₈-Alkyl;
X Hal; ein Rest der Formel 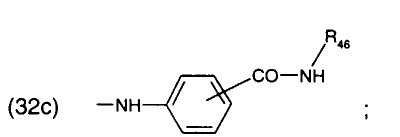 oder 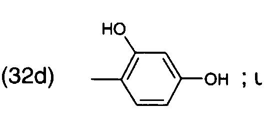 und
n 0 oder 1;
bedeuten.

14. Verwendung nach Anspruch 1 oder 2, dadurch gekennzeichnet, dass die organischen UV-Filter ausgewählt sind aus Verbindungen der Formel 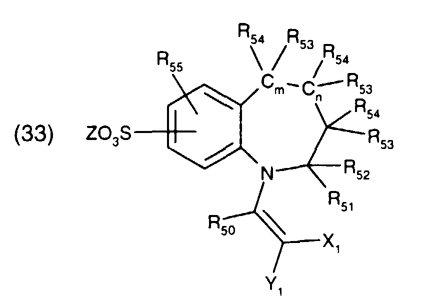 worin
R₅₀, R₅₁, R₅₂, R₅₃, R₅₄ unabhängig voneinander Wasserstoff, C₁-C₈-Alkyl oder C₅-C₁₀-Cycloalkyl;
R₅₅ Wasserstoff; C₁-C₈-Alkyl; C₅-C₁₀-Cycloalkyl; Hydroxy; C₁-C₈-Alkoxy; COOR₅₆; oder CONR₅₇R₅₈;
R₅₆, R₅₇ und R₅₈ unabhängig voneinander Wasserstoff oder C₁-C₆-Alkyl;
X und Y unabhängig voneinander Wasserstoff, -CN; CO₂R₅₉; CONR₅₉R₆₀; oder COR₅₉;
wobei die Reste X und Y zusätzlich einen C₁-C₈-Alkylrest, ein C₅-C₁₀-Alkylrest oder einen Heteroarylrest mit 5 bis 6 Ringatomen sein können, wobei ferner X und Y oder
R₅₀ zusammen mit einem der Reste X und Y den Rest zur Vervollständigung eines 5- bis 7-gliedrigen Ringes bedeuten kann, der bis zu 3 Heteroatome enthalten kann, wobei die Ringatome mit exocyclisch doppelt gebundenem Sauerstoff und/oder C₁-C₈-Alkyl- und/oder C₅-C₁₀-Cycloalkylresten substituiert sein können und/oder C=C-Doppelbindungen enthalten können;
Z Wasserstoff; Ammonium; Alkalimetallion; oder das Kation einer zur Neutralisation der freien Säuregruppe eingesetzte organische Stickstoffbase,
R₅₉ und R₆₀ unabhängig voneinander Wasserstoff, C₁-C₈-Alkyl oder C₅-C₁₀-Cycloalkyl; und
n und m unabhängig voneinander 0 oder 1 bedeuten.

15. Verwendung nach einem der Ansprüche 1 bis 14, dadurch gekennzeichnet, dass die organischen UV-Filter als Mischungen eingesetzt werden.

16. Verfahren zur Herstellung von Mischungen der in einem der Ansprüche 1 bis 14 definierten erfindungsgemäss einsetzbaren organischen UV-Filter, dadurch gekennzeichnet, dass man die in mikronisierter Form vorliegenden UV-Filter innig miteinander vermischt.

17. Verfahren zur Herstellung von Mischungen der in einem der Ansprüche 1 bis 14 definierten erfindungsgemäss einsetzbaren organischen UV-Filter, dadurch gekennzeichnet, dass die organischen UV-Filter als Gemische von mindestens zwei Einzelsubstanzen mikronisiert werden.

18. Verfahren zur Herstellung von Mischungen der in einem der Ansprüche 1 bis 14 definierten erfindungsgemäss einsetzbaren organischen UV-Filter, dadurch gekennzeichnet, dass mindestens zwei Einzelsubstanzen zusammengeschmolzen werden, die Schmelze abgekühlt, und der entstandene Composite anschliessend einem Mikronisierungsprozess unterworfen wird.

19. Composite, erhältlich durch Zusammenschmelzen von mindestens zwei der in einem der Ansprüche 1 bis 14 definierten organischen UV-Filter.

20. Verwendung nach einem der Ansprüche 1 bis 14, dadurch gekennzeichnet, dass zusätzlich ein anorganisches Pigment hinzugemischt wird.

21. Verwendung nach Anspruch 20, dadurch gekennzeichnet, dass die anorganischen Pigmente ausgewählt sind aus TiO₂, ZnO, Eisenoxiden, Glimmer (Mica) und Ti- oder Zinksalze von organischen Säuren.

22. Composite, erhältlich durch Zusammenschmelzen von mindestens zwei der in einem der Ansprüche 1 bis 14 definierten organischen UV-Filter und mindestens einem der in Anspruch 20 oder 21 definierten anorganischen Pigmente.

23. Verwendung nach einem der Ansprüche 1 bis 14, dadurch gekennzeichnet, dass zusätzlich ein Antioxidans hinzugemischt wird.

24. Verwendung nach Anspruch 23, dadurch gekennzeichnet, dass das Antioxidans ausgewählt ist aus Tocopherolen, Ellagsäure, Propylgallat, butyliertem Hydroxytoluol, butyliertem Hydroxyanisol, 2,4,6-Tris(3,5-di-t-butyl-4-hydroxybenzyl)mesitylen, Tetrakis-[methylen-3(3',5'-di-t-butyl-4'-hydroxyphenyl)propionat]methan, der Verbindung der Formel 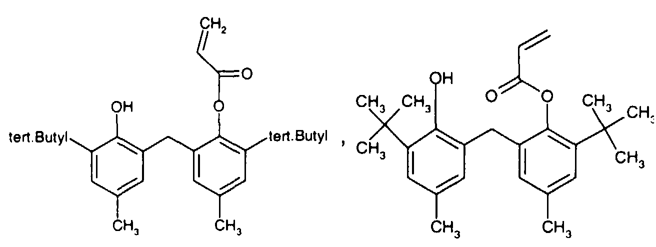 oder 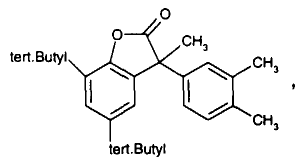 Vanillin, Ubichinon, Ferulasäure, Ferulasäurederivaten, Rutinsäure, Rutinsäurederivaten; Urocaninsäure, Urocaninsäurederivaten und Propolis.

25. Composite, erhältlich durch Zusammenschmelzen von mindestens zwei der in einem der Ansprüche 1 bis 14 definierten organischen UV-Filter und mindestens einem der in Anspruch 23 oder 24 definierten Antioxidantien und gegebenenfalls einem oder mehreren anorganischen Pigmenten.

26. Verwendung nach einem der Ansprüche 1 bis 15, dadurch gekennzeichnet, dass eine kationische oder anionische Verbindung hinzugemischt wird.

27. Verwendung nach Anspruch 26, dadurch gekennzeichnet, dass die kationische oder anionische Verbindung ausgewählt ist aus Campherbenzalkoniummethosulfaten, Fettaminen, Betainen, Quats, Zitronensäuremonoglycerid, Natriummethylcocoyltaurat, Phospholipiden, Ceramiden und Phytosterolen.

28. Composite, erhältlich durch Zusammenschmelzen von mindestens zwei der in einem der Ansprüche 1 bis 14 definierten organischen UV-Filter und mindestens einem der in den Ansprüchen 26 und 27 definierten kationischen bzw. anionischen Verbindungen.

29. Verwendung nach einem der Ansprüche 1 bis 15, dadurch gekennzeichnet, dass zusätzlich ein pharmazeutischer oder kosmetischer Wirkstoff hinzugemischt wird.

30. Kosmetische Formulierung, enthaltend einen der in einem der Ansprüche 1 bis 15 definierten organischen UV-Filter, gegebenenfalls einen oder mehrere Antioxidantien und/oder anorganische Pigmente und/oder eine kationische bzw. anionische Verbindung, sowie kosmetisch verträgliche Träger- oder Hilfsstoffe.

31. Kosmetische Formulierung nach Anspruch 30, dadurch gekennzeichnet, dass sie zusätzlich einen öllöslichen, nicht mikronisierten UV-Filter enthält.

32. Pharmazeutische Formulierung, enthaltend ein Gemisch aus mindestens zwei der in einem der Ansprüche 1 bis 15 definierten organischen UV-Filter, gegebenenfalls einen oder mehrere Antioxidantien und/oder anorganische Pigmente und/oder eine kationische bzw. anionische Verbindung, sowie pharmazeutisch verträgliche Träger- oder Hilfsstoffe.
